# EUROPEAN PATENT APPLICATION

(11) **EP 2 415 746 A1**
(43) Date of publication of application: **08.02.2012**
(21) Application number: 10758602.6
(22) Date of filing: 29.03.2010
(51) Int. Cl.: C07C 49/67, C07D 307/91, C09K 11/06, H01L 51/50, H05B 33/10

(54) **LIGHT-EMITTING ELEMENT MATERIAL PRECURSOR BODY AND PRODUCTION METHOD THEREFOR**

(30) Priority: 31.03.2009 JP 2009085785
(71) Applicant: Toray Industries, Inc., Tokyo 103-8666 (JP)
(72) Inventor: SHIRASAWA, Nobuhiko, Otsu-shi Shiga 520-8558 (JP); JO, Yukari, Otsu-shi Shiga 520-8558 (JP); FUJIMORI, Shigeo, Otsu-shi Shiga 520-8558 (JP)
(74) Representative: Hager, Thomas Johannes
(86) International application number: PCT/JP2010/055502
(87) International publication number: WO 2010/113831

(57) **Abstract**

Disclosed are a method which can produce a chemical compound precursor body for a light-emitting element material which is difficult to produce using conventional methods, and a method for therefrom producing a suitable material and an organic electroluminescent element utilizing same. The disclosed precursor body for a light-emitting element material is represented by general formula 1. [Formula 1] (wherein, R1 - R6 may be the same or different from each other, and are chosen from the set consisting of hydrogen, an alkyl group, a cycloalkyl group, an alkenyl group, a cycloalkenyl group, an alkoxy group, an alkylthio group, an aryl ether group, an aryl thieoether group, an aryl group, a heteroaryl group, and a halogen. However, of R¹ - R⁶, at least one must have a condensed aromatic hydrocarbon with at least two rings.)

## Description

### TECHNICAL FIELD

The present invention relates to a precursor of a light emitting device material useful as a constituent material of an organic electroluminescence (hereinafter referred to as "EL") element, a method for producing the precursor, and a light emitting device using it. The technical field especially includes light emitting device materials which can be used for the fields of display elements, flat panel displays, backlights, illuminations, interior, signs, signboards, electrophotographic devices, optical signal generators and the like.

### BACKGROUND ART

In recent years, research on organic EL elements, wherein electrons injected from a cathode and positive holes injected from an anode are recombined in an organic fluorescent body between the both electrodes to emit light, has been actively carried out. These light emitting devices are drawing attention since they are thin and capable of high-intensity emission under low driving voltage, and multicolor emission is possible by selecting the light emitting device materials.

The methods for preparation of organic EL elements can be grouped into the following two types: the dry process represented by the vacuum deposition method; and the wet process by the spin coating method or the ink jet method.

In the vacuum deposition, a low-molecular material is sublimated in vacuum to achieve film formation on a device substrate. By this method, a device wherein plural types of materials are layered with controlled desired thicknesses can be prepared (see Non-patent Document 1). By this production method, practical and high-performance organic EL elements can be obtained.

On the other hand, in the vacuum deposition method, there are problems in that the production apparatus is expensive and the ratio of the material with which the film is formed on the substrate is low with respect to the material used. Further, in the patterning method using a shadow mask, preparation of large-area devices is difficult.

In the wet process represented by the spin coating method, the ink jet method and the nozzle coating method, a large-area device, which can achieve improved utilization rate of the material and patterning, can be easily prepared, so that the difficulties in the vacuum deposition method can be overcome, which is advantageous (see Non-patent Document 2). However, since only a material having sufficient solubility in a solvent suitable for the process can be used, it is difficult to use the low molecular material as it is, which material realizes a high-performance organic EL element. Therefore, at present, an organic EL element prepared by the wet process often shows a lower element performance compared to one prepared by the vacuum deposition method

On the other hand, a technology has been developed in which a solution containing a soluble precursor is applied to a device substrate to carry out film formation, and heat treatment is then carried out to achieve conversion to an organic semiconductor material, thereby obtaining a property of interest (see Patent Documents 1 to 3). Since, in this method, even in cases where the material has poor solubility, its precursor is soluble, so that the material can be applied to the wet process.

### PRIOR ART DOCUMENTS

### Patent Documents

[Patent Document 1] JP 2003-304014 A (US 2003-226996 A)
[Patent Document 2] JP 2005-232136 A
[Patent Document 3] JP 2008-135198 A

### Non-patent Documents

[Non-patent Document 1] "Applied Physics Letters", 1987, 51(12), pp. 913-915.
[Non-patent Document 2] "2007 SID International Symposium DIGEST OF TECHNICAL PAPERS", pp. 1834-1837.

### SUMMARY OF THE INVENTION

### PROBLEMS TO BE SOLVED BY THE INVENTION

However, in the methods described in Patent Documents 1 to 3, the compound for which a soluble precursor can be produced is restricted. For example, the methods could not be applied to an anthracene derivative having substituents at positions 9 and 10, a pyrene derivative, and a 4-substituted tetracene derivative represented by rubrene.

Thus, there have been materials which could not be applied to the element by the wet process according to conventional technologies. To solve these problems, the present invention aims to enable production of a soluble precursor of a light emitting device material (hereinafter referred to as "light emitting device material precursor") which could not be conventionally obtained, and to thereby provide a material suitable for the wet process and a method for producing an organic EL element using the material.

### MEANS FOR SOLVING THE PROBLEMS

In order to solve the above-mentioned problems, the present invention has the following contents.

First, the present invention provides a light emitting device material precursor represented by the Formula (1):

[Chemical Formula 1]

(wherein R¹ to R⁶ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups and heteroaryl groups, with the proviso that at least one of R¹ to R⁶ has a fused aromatic hydrocarbon having two or more rings).
The present invention further provides a process of producing the above-described light emitting device, which process has the following constitution:
A process of producing a bicyclo-[2,2,2]-cyclooctadiene-2,3-dione derivative, comprising the steps of obtaining a compound represented by the Formula (8) from a compound represented by the Formula (7) below by a reaction replacing R¹⁷¹;
eliminating protective groups in the obtained compound represented by the Formula (8) to obtain a compound represented by the Formula (9); and converting the compound represented by the Formula (9) to a compound represented by the Formula (10) by an oxidation reaction of the compound represented by the Formula (9);
(wherein R¹⁷⁰ is an aryl group or heteroaryl group; R¹⁷¹ is an electron-withdrawing group; R¹⁷² to R¹⁷⁶ are selected from hydrogen, alkyl groups, cycloalkyl groups,
alkenyl groups, cycloalkenyl groups, alkoxy groups, alkylthio groups, arylether groups, aryl thioether groups, aryl groups, heteroaryl groups and halogens; R¹⁷⁷ to R¹⁷⁸ is selected from hydrogen, alkyl groups, alkoxy groups, aryl groups and arylether groups).

[Chemical Formula 2]

### EFFECTS OF THE INVENTION

By the present invention, light emitting device material precursors of various light emitting device materials, which could not be produced by conventional methods, can be produced. Further, by applying a solution containing the light emitting device material precursor and forming a film therewith by the ink jet method or the nozzle coating method, followed by treatment for conversion to a device constituent material, a device can be produced.

### BRIEF DESCRIPTION OF THE DRAWINGS

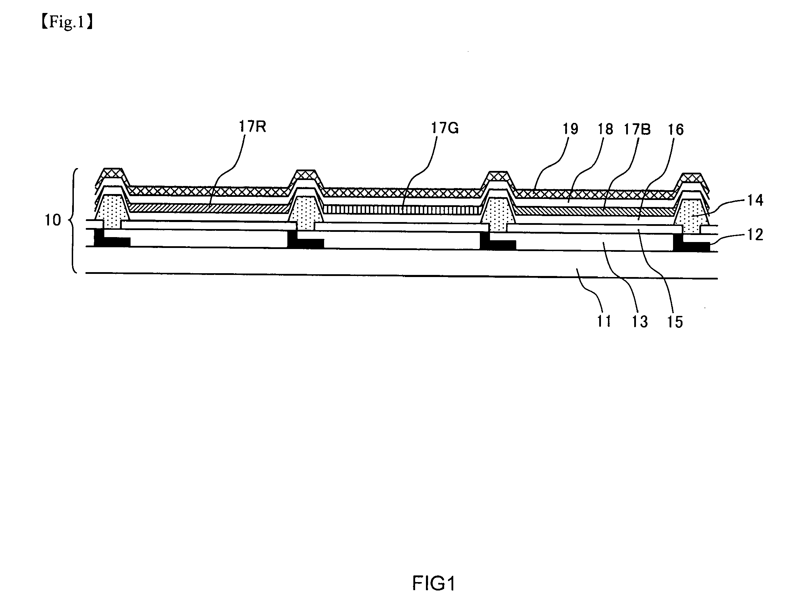

Fig. 1 is a cross-sectional view showing an example of an organic EL element wherein emitting layers are patterned by the present invention.

### MODE FOR CARRYING OUT THE INVENTION

The light emitting device material precursor of the present invention is represented by the Formula (1):

[Chemical Formula 3]

(wherein R¹ to R⁶ each may be the same or different and is selected from hydrogen, alkyl groups, cycloalkyl groups, alkenyl groups, cycloalkenyl groups, aryl groups, alkoxy groups, arylether groups, alkylthio groups, aryl thioether groups, heteroaryl groups and halogens, with the proviso that at least one of R¹ to R⁶ has a fused aromatic hydrocarbon having two or more rings).

Preferred light emitting device material precursors are represented by Formula (1'):

[Chemical Formula 4]

wherein R¹ to R⁶ have the same meanings as in Formula (1), with the proviso that at least one of R¹ to R⁶ has a fused aromatic hydrocarbon having three or more rings.

The light emitting device material precursor represented by the Formula (1) is [2,2,2]-bicyclooctadiene-2,3-dione to which R¹ to R⁶ are bound. The light emitting device material precursor described below also has [2,2,2]-bicyclooctadiene-2,3-dione as the skeleton. These light emitting device material precursors liberate two molecules of carbon monoxide by light irradiation and change the structure to convert to light emitting device materials. The [2,2,2]-bicyclooctadiene-2,3-dione converts to a benzene ring with the substituents remain attached to the carbons. For example, as shown in the scheme below, it liberates two molecules of carbon monoxide by light irradiation (indicated by "hv" in the scheme) and changes its structure to convert to a light emitting device material. Further, since the light emitting device material of the present invention has a bulky bicyclo structure moiety in its molecule, it can suppress aggregation of the molecules, so that the solubility in solvents is increased.

[Chemical Formula 5]

The present invention is especially effective when a light emitting device material having a fused aromatic hydrocarbon is desired. That is, fused aromatic hydrocarbons having a large number of fused rings have a low polarity of the molecule, so that the affinity to polar solvents is low. By incorporating [2,2,2]-bicyclooctadiene-2,3-dione which is a characteristic chemical structure to the present invention, the polarity of the molecule is increased, so that the affinity to the polar solvents is increased and becomes easier to dissolve. Further, as mentioned above, the effect to suppress the aggregation of the molecules by the bulky bicyclo structure is especially effective for increasing the solubility in non-polar solvents of the compounds having a fused aromatic hydrocarbon having a large number of the fused rings.

By the present invention, light emitting device material precursors of the compounds such as anthracene derivatives and pyrene derivatives having substituents at 9-position or 10-position can be produced. Therefore, the compounds suitable as light emitting device materials can be produced by a wet process.

An alkyl group means a saturated aliphatic hydrocarbon group, such as methyl group, ethyl group, n-propyl group, isopropyl group, n-butyl group, sec-butyl group or tert-butyl group, and it may or may not have a substituent(s). In the present invention, the alkyl group preferably has 1 to 20 carbon atoms. In cases where the alkyl group has a substituent(s), the substituent is not restricted and examples thereof include alkyl groups, aryl groups and heteroaryl groups.
A cycloalkyl group means a saturated alicyclic hydrocarbon group, such as cyclopropyl group, cyclohexyl group, norbornyl group or an adamantyl group. In the present invention, the cycloalkyl group preferably has 3 to 20 carbon atoms.
The cycloalkyl group may or may not have a substituent(s).

An alkenyl group means an unsaturated aliphatic hydrocarbon group containing a double bond, such as vinyl group, allyl group or butadienyl group. In the present invention, the alkenyl group preferably has 2 to 20 carbon atoms. The alkenyl group may or may not have a substituent(s).

A cycloalkenyl group means an unsaturated alicyclic hydrocarbon group containing a double bond, such as cyclopentenyl group, cyclopentadienyl group or cyclohexenyl group. In the present invention, the cycloalkenyl group preferably has 3 to 20 carbon atoms, and it may or may not have a substituent(s).
An aryl group means an aromatic hydrocarbon group or a group in which a plurality of aromatic hydrocarbon groups are bound, such as phenyl group, naphthyl group, biphenyl group, fluorenyl group, phenanthryl group, terphenyl group, anthracenyl group or pyrenyl group. In the present invention, the aryl group preferably has 6 to 40 carbon atoms. The aryl group may or may not have a substituent(s). Examples of the substituent which the aryl group may have include alkyl groups, cycloalkyl groups, alkenyl groups, alkynyl groups, alkoxy groups, arylether groups, alkylthio groups, halogens, cyano group, amino group, silyl group and boryl group.

An alkoxy group means a functional group wherein aliphatic hydrocarbon groups are bound via an ether bond, such as methoxy group, ethoxy group or propoxy group. In the present invention, the alkoxy group preferably has 1 to 20 carbon atoms. The aliphatic hydrocarbon groups may or may not have a substituent(s).

An arylether group means a functional group wherein aromatic hydrocarbon groups are bound via an ether bond, such as phenoxy group. In the present invention, the arylether group preferably has 6 to 40 carbon atoms. The aromatic hydrocarbon groups may or may not have a substituent(s).

An alkylthio group means a group wherein the oxygen atom in the ether bond in an alkoxy group is replaced by a sulfur atom. In the present invention, the alkylthio group preferably has 1 to 20 carbon atoms. The hydrocarbon group in the alkylthio group may or may not have a substituent(s).

An aryl thioether group is a group wherein the oxygen atom in the ether bond in an arylether group is replaced by a sulfur atom. In the present invention, the aryl thioether group preferably has 1 to 20 carbon atoms. The aromatic hydrocarbon groups in the aryl thioether group may or may not have a substituent(s).

A heteroaryl group means an aromatic group having an atom(s) other than carbon atom in a ring, such as furanyl group, thiophenyl group, oxazolyl group, pyridyl group, quinolinyl group or carbazolyl group. In the present invention, the heteroaryl group preferably has 2 to 30 carbon atoms. The aromatic group may or may not have a substituent(s).

Halogen means fluorine, chlorine, bromine, iodine or the like.

A fused aromatic hydrocarbon means an aromatic compound composed of carbon and hydrogen having a structure in which two rings share one side of a benzene ring, such as naphthalene. Examples thereof include naphthalene, anthracene, triphenylene, phenanthrene, tetracene, pyrene, chrysene, pentacene, perylene and coronene.

The R¹ to R⁶ other than the group(s) having a fused aromatic hydrocarbon group having two or more rings are, among the above-described substituents, preferably aryl groups or hydrogen, and especially preferably, all of them are hydrogen. As described above, the diketo crosslinking unit represented by the Formula (1) forms a benzene ring by the liberation of carbon monoxide. Therefore, in cases where all of the R¹ to R⁶ other than the group(s) having a fused aromatic hydrocarbon group having two or more rings are hydrogen, the diketo bridging unit is converted to phenyl group. That is, a compound which is a derivative of a fused aromatic hydrocarbon compound, which has a phenyl group at its terminal can be obtained.

In view of the light emitting properties of the light emitting device material, in the light emitting device material precursor of the present invention, it is preferred that at least one of R¹ to R⁶ in the Formula (1) or Formula (1') contain a skeleton represented by the Formulae (2-1) to (2-7):

[Chemical Formula 6]

In Formulae (2-1) to (2-7), R¹⁰ to R¹⁷, R²⁰ to R²⁷, R³⁰ to R³⁷, R⁴⁰ to R⁴⁹, R⁵⁰ to R⁶¹, R⁷¹ to R⁸¹ and R⁸² to R⁸⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused rings formed between adjacent substituents.

X¹ to X⁶, Y¹ to Y⁴ and Z¹ to Z² each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused rings formed between adjacent substituents.

Provided, however, that in Formula (2-1), the cases where X¹ and X² are hydrogen are excluded; that, in Formula (2-2), the cases where X³ and X⁴ are hydrogen, and the cases where Y¹ and Y² are hydrogen are excluded; and that, in Formula (2-3), the cases where X⁵ and X⁶ are hydrogen, the cases where Y³ and Y⁴ are hydrogen, and the cases where Z¹ and Z² are hydrogen are excluded.

At least one of R¹⁰ to R¹⁷ and X¹ to X² in Formula (2-1), at least one of R²⁰ to R²⁷, X³ to X⁴ and Y¹ to Y² in Formula (2-2), at least one of R³⁰ to R³⁷, X⁵ to X⁶, Y³ to Y⁴ and Z¹ to Z² in Formula (2-3), at least one of R⁴⁰ to R⁴⁹ in Formula (2-4), at least one of R⁵⁰ to R⁶¹ in Formula (2-5), at least one of R⁷⁰ to R⁸¹ in Formula (2-6), and at least one of R⁸² to R⁸⁹ in Formula (2-5), respectively, is used to bind to the diketo bridging unit in Formula (1) through direct bond or through a linking group).
A heterocyclic group means an aliphatic ring having an atom(s) other than carbon, such as pyran ring, piperidine ring or cyclic amide. In the present invention, the heterocyclic group preferably has 2 to 20 carbon atoms.

An alkynyl group means an unsaturated aliphatic hydrocarbon group containing a triple bond, such as ethynyl group, and this may or may not have a substituent(s). In the present invention, the alkynyl group preferably has 2 to 20 carbon atoms.

A carbonyl group means a substituent having a carbon-oxygen double bond, such as an acyl group or formyl group. An acyl group means a substituent wherein the hydrogen in formyl group is substituted by an alkyl group, an aryl group or a heteroaryl group. An oxycarbonyl group means a substituent having an ether bond on the carbon atom in carbonyl group, such as t-butyloxycarbonyl group or benzyloxycarbonyl group. In the present invention, the carbonyl group preferably has 1 to 20 carbon atoms.
A carbamoyl group means a substituent wherein the hydroxyl group in carbamic acid is eliminated, and it may or may not have a substituent(s). In the present invention, the carbamoyl group preferably has 1 to 20 carbon atoms.

An amino group means a nitrogen compound group such as dimethylamino group, and it may or may not have a substituent(s). In the present invention, the amino group preferably has 0 to 20 carbon atoms.

A silyl group means a silicon compound group such as trimethylsilyl group, and it may or may not have a substituent(s). In the present invention, the silyl group preferably has 1 to 20 carbon atoms.

A phosphineoxide group means a substituent containing a phosphorus-oxygen double bond, and it may or may not have a substituent(s). In the present invention, the silyl group preferably has 1 to 20 carbon atoms.

A fused ring formed between adjacent substituents means, when explained referring to the above-described Formula (2-1) as an example, the conjugate or non-conjugate fused ring formed by binding of arbitrary two adjacent substituents (e.g., R¹⁰ and R¹¹) selected from R¹⁰ to R¹⁷. The fused ring may contain a nitrogen, oxygen or sulfur atom in the ring structure, and may be fused with another ring.

Among the light emitting device material precursors of the present invention, in view of the light emitting properties and durability, those represented by Formula (3) or (4) are preferred:

[Chemical Formula 7]

wherein R⁹⁰ to R⁹⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents; R¹⁰⁰ to R¹⁰⁵ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens; A is selected from single bond, arylene groups and heteroarylene groups; m is an integer of 1 to 3; some of R⁹⁰ to R⁹⁹ in the number of m is used to bind to A; and at least one of R¹⁰⁰ to R¹⁰⁵ is used to bind to A.

[Chemical Formula 8]

wherein R¹¹⁰ to R¹¹⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents. R¹²⁰ to R¹²⁵ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens. B is selected from single bond, arylene groups and heteroarylene groups. n is an integer of 1 to 2. Some of R¹²⁰ to R¹²⁵ in the number of n and some of R¹¹⁰ to R¹¹⁹ in the number of n are used to bind to B. At least one of R¹²⁰ to R¹²⁵ is used to bind to B.
An arylene group herein means a divalent group derived from an aromatic hydrocarbon group such as phenyl group, naphthyl group, biphenyl group, phenanthryl group, terphenyl group or pyrenyl group, and it may or may not have a substituent(s). In the present invention, the arylene group preferably has 6 to 40 carbon atoms.

An heteroarylene group means a divalent group derived from an aromatic group having an atom(s) other than carbon, such as furanyl group, thiophenyl group, oxazolyl group, pyridyl group, quinolinyl group or carbazolyl group, and it may or may not have a substituent(s). In the present invention, the heteroarylene group preferably has 2 to 30 carbon atoms.

Preferred examples of the arylene group and heteroarylene group include, but are not limited to, those shown below.

[Chemical Formula 9]

As the light emitting device material precursors of the present invention, those represented by Formula (5) or (6) are preferred as the materials of the resulting organic EL elements.

[Chemical Formula 10]

wherein R¹³⁰ to R¹³⁸ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents. R¹⁴⁰ to R¹⁴⁴ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens.

[Chemical Formula 11]

wherein R¹⁵⁰ to R¹⁵⁸ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl groups, carboxyl group, oxycarbonyl group, carbamoyl group, amino group, silyl group, phosphineoxide group and the fused ring formed between adjacent substituents. R¹⁶⁰ to R¹⁶⁴ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens.

By using the compound represented by Formula (5) or (6), pyrene derivatives having a phenyl group and anthracene derivatives having a phenyl group at 9-position or 10-position can be obtained. Here, the phenyl group is preferably one which is not substituted. Since most of the light emitting device materials converted from a light emitting device material precursor wherein at least one of R¹³⁰ to R¹³⁸ in Formula (5) or at least one of R¹⁵⁰ to R¹⁵⁸ in Formula (6) is an aryl group, a heteroaryl group or an alkenyl group have a poor solubility due to the π-π interaction, the precursors of the present invention exhibit an especially great advantage when applied to these compounds. Among those described above, those having an aryl group or a heteroaryl group are preferred. The compounds represented by Formula (5) are converted to light emitting device materials wherein the [2,2,2]-bicyclooctadiene-2,3-dione structure changes to a benzene ring by light irradiation remaining the binding state of R¹⁴⁰ to R¹⁴⁴. The compounds represented by Formula (6) are converted to light emitting device materials wherein the [2,2,2]-bicyclooctadiene-2,3-dione structure changes to a benzene ring by light irradiation remaining chemical bonds of R¹⁶⁰ to R¹⁶⁴.
Specific examples of the light emitting device material obtained from the light emitting device material precursors of the present invention include those described in the prior art references below. In other words, as the light emitting device material precursors of the present invention, those which can be converted to the materials described in the patent documents described below are preferably employed.

The following Japanese patent documents:
JP 2006-042091 A (JP 3196230 B), JP 2002-8867 A, JP 2006-190759 A, JP 2006-245172 A, JP 2006-265515 A, JP 2007-131722 A, JP 2007-131723 A, JP 2007-201491 A, JP 2008-124157 A, JP 2008-159843 A, JP 2008-7785 A, JP 2008-252063 A, JP 2009-10408 A, JP 2009-049094 A; and
   the following international publication pamphlets:
WO2004/096945, WO2005/115950, WO2007/029798, WO2007/097178, WO2008/108256.

The light emitting device material precursors of the present invention are preferably employed, when the compounds, among these compounds, wherein one of R¹³¹, R¹³⁴ and R¹³⁶ is a substituent containing a carbazole skeleton, dibenzothiophene skeleton or dibenzofuran skeleton, or the compounds wherein R¹⁵⁴ is a substituent containing a carbazole skeleton, dibenzothiophene skeleton or dibenzofuran skeleton, are used. More preferably precursors are those containing a carbazole skeleton or dibenzofuran skeleton, that is, those represented by Formula (5') or (6').

[Chemical Formula 12]

wherein R¹⁸⁰ to R¹⁸⁷ and R¹⁹⁰ to ¹⁹⁷ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogen, cyano group, carbonyl group, carboxyl group, oxycarbonyl group, carbamoyl group, amino group, silyl group, phosphineoxide group and fused ring formed between adjacent substituents. R¹⁸⁸ is selected from hydrogen, alkyl groups, cycloalkyl groups, heterocyclic groups, alkenyl groups, cycloalkenyl groups, alkynyl groups, aryl groups, heteroaryl groups, carbonyl group, carboxyl group, oxycarbonyl group, carbamoyl group, silyl group and phosphineoxide group.

The compounds represented by Formula (5') are converted to light emitting device materials wherein the [2,2,2]-bicyclooctadiene-2,3-dione structure changes to a benzene ring by light irradiation remaining the binding state of R¹⁴⁰ to R¹⁴⁴. The compounds represented by Formula (6') are converted to light emitting device materials wherein the [2,2,2]-bicyclooctadiene-2,3-dione structure changes to a benzene ring by light irradiation remaining the binding state of R¹⁶⁰ to R¹⁶⁴.
These compounds are preferably employed since a higher efficiency of the light emitting device material of an organic EL element is obtained after the conversion of the skeleton.

Examples of the structural formula of the preferred compounds as the light emitting device material precursors of the present invention are described below. However, the present invention is not limited to these compounds, but any compound which can be converted to one of those described above is preferred. Among these, those which can be converted to the compounds described in the pamphlets, Japanese patent documents or the like are preferred. W02005/113531, WO2007/29798, WO2008/108256, JP 2007-131722 A, JP 2007-131723 A and so on.

First, those having the following chemical structures are shown. This group is hereinafter referred to as "Group A".

[Chemical Formula 13]

Further, those having the following chemical structures are shown. This group is hereinafter referred to as "Group B".

[Chemical Formula 14]

Still further, those having the following chemical structures are shown. This group is hereinafter referred to as "Group C".

[Chemical Formula 15]

The light emitting device material precursors having the structures exemplified in Groups A, B and C are converted to light emitting device materials wherein the [2,2,2]-bicyclooctadiene-2,3-dione structure is converted to a benzene ring by light irradiation.

As the light emitting device material precursors of the present invention, those with which a solution having a concentration of not less than 0.5% by weight, more preferably not less than 2% by weight, can be prepared under atmospheric pressure with any one of the solvents used in the coating process are preferred.

The solvent for constituting the solution is not restricted and those with which the solution having the above-described concentration can be prepared and which have the boiling point, coefficient of viscosity and surface tension suited for the coating process are preferred. Specific examples include, but are not limited to, water, alcohols having a boiling point of not lower than 100°C and not higher than 250°C (e.g., cyclohexanol, benzyl alcohol, octanol, trimethylhexanol, ethylene glycol and the like), chloroform, chlorobenzene, dichlorobenzene, trichlorobenzene, toluene, xylene, benzoic acid esters, tetralin (tetrahydronaphthalene), decalin (decahydronaphthalene), propionitrile, benzonitrile, acetophenone, cyclohexanone, phenol, γ-butyrolactone, N-methyl-2-pyrrolidone and 1,3-dimethyl-2-imidazolidinone. Mixtures of a plurality of these solvents may also be used. It is preferred to use a solvent having a purity as high as possible because the characteristic of the produced device may be degraded by the impurities contained in the solvents.

The solution for the coating can be prepared by placing the precursor material and the solvent in a vessel, and stirring the mixture. In this step, the dissolution can be accelerated by heating or by ultrasonication, and magnetic stirrer or a mechanical stirrer may be used as stirring means.

The process of producing the light emitting device material precursor represented by Formula (1) is now described. The bicyclo-[2,2,2]-cyclooctadiene-2,3-dione derivatives which are the light emitting device material precursors of the present invention can be produced by the process described below.

That is, the derivatives can be produced by the process of producing a bicyclo-[2,2,2]-cyclooctadiene-2,3-dione derivative, comprising the steps of obtaining a compound represented by the Formula (8) from a compound represented by the Formula (7) below by a reaction replacing R¹⁷¹ ; eliminating R¹⁷⁷ to R¹⁷⁸ which are protective groups in the obtained compound represented by the Formula (8) to obtain a compound represented by the Formula (9); and converting the compound represented by the Formula (9) to a compound represented by the Formula (10) by an oxidation reaction of the compound represented by the Formula (9)

[Chemical Formula 16]

wherein R¹⁷⁰ is an aryl group or heteroaryl group, and is preferably a group having a fused aromatic hydrocarbon group having two or more fused rings, more preferably one containing an anthracene skeleton or pyrene skeleton, still more preferably one having a structure represented by one of the above-described Formulae (2-1) to (2-7). R¹⁷¹ is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and substituted sulfonyl groups. Among these, an electron-withdrawing group is preferably employed. R¹⁷² to R¹⁷⁶ are selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens. R¹⁷⁷ to R¹⁷⁸ is selected from hydrogen, alkyl groups, alkoxy groups, aryl groups and arylether groups.

The substituted sulfonyl group herein means a monovalent substituent wherein one substituent is introduced into sulfonyl group, and examples thereof include p-toluenesulfonyl group, trifluoromethanesulfonyl group, benzenesulfonyl group and alkylsulfonyl groups.

Examples of the electron-withdrawing group herein include carbonyl group, fluorine-substituted alkyl groups, fluorine-substituted aryl groups, heteroaryl groups (those substituted with fluorine are preferred), carboxyl group, carbamoyl group and substituted sulfonyl group.

The intermediate (7) can be synthesized by the Diels-Alder reaction between the acetylene derivative (11) and the acetonide (12) of 3,5-cyclohexadiene-1,2-diol derivative. If R¹⁷¹ is an electron-withdrawing group, the intermediate (7) can be synthesized with a high yield.

[Chemical Formula 17]

The solvent in this reaction is not restricted as long as it dissolves the reactants, and examples thereof include the solvents used for the above-described light emitting device material precursors. Among these, it is preferred to use an aromatic hydrocarbon such as toluene or benzene. The reaction temperature is preferably 20°C to 180°C, more preferably 50°C to 90°C. In cases where the reaction temperature is higher than the boiling point of the solvent used, the reaction mixture may be heated in a closed system by using a pressure container such as an autoclave. In cases where one having a halogen as R¹⁷² to R¹⁷⁵ is used as the acetonide (12) of the 3,5-cyclohexanediene-1,2-diol derivative, dehalogenation may be performed before proceeding to the next step. Examples of the halogenation method include hydrogenation reaction using palladium catalyst or the like; methods wherein the acetonide (9) is subjected to lithiation or converted to an organic metal compound such as Grignard reagent and then the resultant is hydrolyzed; and methods by a reducing agent such as sodium amalgam.

In cases where R¹⁷⁶ is hydrogen, the production of the intermediate (8) can be attained by treating the intermediate (7) with an appropriate reducing agent. Examples of the method of attaining this include hydrogenation reaction using palladium as a catalyst; reduction reaction by an amalgam; and reduction reaction by using a mixture system of samarium iodide/hexamethylphosphoric triamide (HMPA). Among these, in view of the safety and simplicity of the reaction, it is preferred to conduct the method by the reduction reaction by using samarium iodide/HMPA mixed system. Any solvent can be used as the reaction solvent used here without restriction as long as it does not react with the reducing agent, and diethyl ether or tetrahydrofuran is preferably employed. The reaction temperature is preferably from -50°C to 30°C. In cases where R¹⁷⁶ is one other than hydrogen, the method can be attained by the method described in "Tetrahedron Letters", 1991, 32, 35, 4583-4586. That is, the intermediate (8) can be obtained by reacting a lithium reagent or Grignard reagent of the desired substituent with the intermediate (7).

The intermediate (9) can be obtained by the reaction to eliminate the protective groups in the intermediate (8). The deprotection reaction is preferably hydrolysis and the catalyst accelerating the reaction is preferably an acid. Examples of the acid used include dilute hydrochloric acid, dilute nitric acid, dilute sulfuric acid, acetic acid, hydrochloric acid-ether complex, pyridinium-p-toluene sulfonate, trifluoroacetic acid, trifluoromethane sulfonic acid and p-toluene sulfonic acid. In cases where an inorganic acid is used as the acid, since the acid is an aqueous solution, it is preferred to use, as the reaction solvent, a solvent such as toluene, diethyl ether, dichloromethane or chloroform, which is separated from water to form two layers. In cases where an organic acid is used as the acid, it is preferred to use, as the solvent, an alcohol such as methanol, ethanol or isopropanol, or toluene, diethyl ether, dichloromethane, chloroform or the like.

The conversion from the intermediate (9) to the desired light emitting device material precursor (10) can be attained by an oxidation reaction using an appropriate oxidizing agent. The oxidation reaction is preferably Swern oxidation using dimethyl sulfoxide as an oxidizing agent. In this reaction, as an activating agent, oxalyl chloride, acetic anhydride, dicyclohexylcarbodiimide or trifluoroacetic anhydride can be used. Among these, trifluoroacetic anhydride with which the desire product can be obtained with a high yield is preferably employed. As the reaction solvent, dimethyl sulfoxide or dichloromethane can be used, and mixture of these can also be used. The reaction temperature is preferably from -90°C to -30°C, more preferably -60°C to -50°C. As a reaction accelerator, a base can be used. As the base, an alkyl amine is preferably used, and a tertiary alkyl amine is more preferably used.

In cases where a compound wherein an arene skeleton further has a substituent(s), such as those shown in the Groups A, B and C described above, is synthesized, a substituted diketone can be synthesized by the above-described method by using as a starting substance an arene compound wherein the site in the arene compound having the desired substituent, to which site the diketo bridged unit is to be introduced, is preliminarily substituted by a halogen such as bromine or iodine. For example, in the synthesis of an anthracene compound having the diketo bridged unit at the 9-position and a carbazolylphenyl group at 10-position, the desired product can be obtained by carrying out the above-described reaction using 9-bromo-10-carbazolylphenylanthracene. A substituted arene compound having a halogen can be synthesized by using as a starting substance a compound wherein a plurality of sites are halogenated, and introducing the desired substituents by cross coupling to the sites other than one site. For example, 9-bromo-10-carbazolylphenylanthracene can be synthesized by the cross coupling reaction between 9,10-dibromoanthracene and equimolarp-(N-carbazolyl)phenylboranic acid ester.

The method of the present invention for producing a light emitting device will now be described. In the following description, the term "device" means a light emitting device.
On a device substrate on which an electrode(s), charge injection layer(s), charge transporting layer(s) and the like were preliminarily layered, the light emitting device material precursor of the present invention is applied by the coating method to carry out film formation, followed by drying the film and carrying out treatment for conversion to a light emitting device material. Thereafter, film formation for the charge transporting layer(s) and the electrode(s) is carried out, thereby completing production of the device.

It is also possible to select a method wherein a donor substrate is prepared by film formation with a light emitting device material precursor, and after conversion treatment, film formation with device materials is carried out by the transfer method on the device substrate.

Among the above-described methods, the method by film formation with a light emitting device material by the transfer method is preferred in view of the efficiency and durability since elution of the bed layer of the device substrate and influence of the residual solvent can be prevented. The case of production of the device by the transfer method is described below in more detail.

The method of the present invention for producing a device comprises:
(1) the applying step, wherein the light emitting device material precursor of the present invention is applied to a donor substrate;
(2) the converting step, wherein the light emitting device material precursor is converted to a light emitting device material; and
(3) the transferring step, wherein the material on the donor substrate is transferred to a device substrate.

In the present invention, when the material is applied to a donor substrate, a light emitting device material precursor which is soluble in the solvent is used, so that the wet process may be employed. Therefore, even a donor substrate having a large area can be easily prepared. Further, since the material is not directly applied to the device substrate, preparation of a multilayer device is not adversely affected by elution of a lower layer upon the application, or the like.

Further, the light emitting device material precursor is converted to a light emitting device material, and transferred onto a device substrate by the transfer. Therefore, even in cases where there is unevenness of application of the material on the donor substrate before the transfer, the unevenness is eliminated upon the transfer, so that an even light emitting device material layer can be formed on the device substrate.
These steps will now be described in order.

### "(1) The applying step, wherein the light emitting device material precursor of the present invention is applied to a donor substrate"

The applying step (1) is a step wherein a material to be transferred to a device substrate is applied on a donor substrate. As the material to be transferred to a device substrate, the light emitting device material precursor described above is used. In general, a light emitting device material is often insoluble in a solvent. On the other hand, since a light emitting device material precursor which is soluble in a solvent is used in the present invention, even a light emitting device material which is insoluble in a solvent can be applied to the present step.

The donor substrate to be used is not restricted as long as it can form a transfer layer by application of a solution containing a light emitting device material precursor and can be used for transfer to a device substrate. When patterning is necessary, compartment patterns (partition walls) may be provided on the donor substrate such that these correspond to the respective patterns. Further, surface treatment may be carried out in order to obtain an excellent film upon coating film formation, or film formation with a material having properties required for the transfer may be preliminarily carried out. Examples of the purpose of the surface treatment include regulation of wettability of the application liquid, and examples of agents for the treatment include silane hydrophilizing agents; and water repellents containing fluorine compounds, such as "Teflon (registered trademark)". Examples of the material having properties required for the transfer include heat-insulating materials, photothermal conversion materials, reflecting materials, drying materials, polymerization initiating materials, polymerization inhibiting materials and insulating materials.

Examples of the method of application of the solution containing a precursor material include the inkjet method, spin coating, blade coating, slit die coating, screen printing coating, nozzle coating, bar coater coating, template coating, the print transfer method, the dip-and-lift method and the spray method. Among these, the inkjet method, the screen printing method, nozzle coating, the print transfer method or the like is preferably used when patterning is required, such as in cases where an organic EL element or an organic transistor element is to be prepared. The film thickness of the light emitting device material precursor is not restricted as long as it is not less than the thickness required for the device material after the conversion, and the film thickness is usually about 20 to 200 nm. The film of the light emitting device material precursor formed by the application preferably does not have pinholes and has an even film thickness.

### "(2) The converting step, wherein the light emitting device material precursor is converted to a device material"

In the converting step (2), the structure is preferably converted by light irradiation. As the irradiation light, light having a peak wavelength of 300 nm or longer is preferably used. In a compound having a skeleton having a strong peak of the absorption band such as π-π* at 300 nm or longer, such as pyrene or anthracene among the light emitting device material precursors of the present invention, use of light having a wavelength included in this absorption band enhances the conversion rate, which is more preferred. The preferred range of the peak wavelength in such cases is 350 to 400 nm. On the other hand, since excitation of the π-π* transition of the diketone portion of the light emitting device material precursor also allows the converting step, light having a peak wavelength within the range of 430 to 470 nm can also be used. In either case, the light to be used preferably has a half bandwidth of the peak of not more than 50 nm.
As the light source for the light irradiation, the combination of a high-intensity light-source lamp and a bandpass filter; light emitting diode; or the like may be used. Examples of the high-intensity light-source lamp include, but are not limited to, high-pressure mercury lamps, halogen lamps and metal halide lamps. Among these, a light emitting diode is preferred since, by using this, light having only the wavelength of interest can be extracted and irradiated.
Further, the content of the light emitting device material precursor in the film after the converting step is preferably less than 5%, more preferably less than 2%. Of course, the content may be not more than the detection limit. This is because the residual light emitting device material precursor may adversely affect the properties of the device.
Some compounds may be converted to light emitting device materials by heat treatment, but the method of the conversion is preferably based on light irradiation in view of suppression of deterioration of the material since conversion under gentle conditions is possible by light irradiation.

The converting step is preferably carried out in an inert gas atmosphere to avoid, as much as possible, the contact with moisture or oxygen, which causes deterioration of the material or decrease in the device properties. Examples of the inert gas atmosphere include noble gas atmospheres such as argon, helium and xenon atmospheres; and nitrogen and carbon dioxide atmospheres. Among these, argon, helium or nitrogen is preferably used, and argon or nitrogen is more preferably used. In the conversion reaction from the precursor in the present invention, carbon monoxide, with which moisture and oxygen contaminated in small amounts in the donor substrate can be removed, is generated during the conversion from the precursor to the light emitting device material, so that a device having excellent durability can be produced. That is, carbon monoxide generated in the thin film is reacted with water and oxygen existing in the thin film by heating and/or light irradiation, and by this, the effect of their removal by conversion to hydrogen, carbon dioxide and/or the like can be expected.

### "(3) The transferring step, wherein the material on the donor substrate is transferred to a device substrate"

The transferring step (3) is a step wherein the device substrate and the donor substrate are superposed on each other and the material on the donor substrate is transferred to the device substrate by heating or light irradiation. As the method of superposition, a known method may be used. Further, for improvement of the efficiency of transfer, the void between the donor substrate and the device substrate is preferably in a vacuum or reduced-pressure atmosphere. However, when necessary, the void may be in an inert gas atmosphere. To make the void between the donor substrate and the device substrate be in a vacuum or reduced-pressure atmosphere, formation of partition walls on the donor substrate using an insulating material to increase adhesion to the device substrate is useful.

The transfer step may be carried out by a known method, and examples of the method include a method wherein the superposed donor substrate and device substrate are heated from the donor substrate side, and a method wherein light is irradiated from the donor substrate side. The heating may be carried out using a hot plate, infrared heater or the like. With a donor substrate on which a photothermal conversion layer is prepared, the same effect as in the case of the heating can be obtained by irradiation of light having a suitable wavelength, and hence the transfer is possible. The light to be irradiated at this time is preferably laser light, whose central wavelength, irradiation intensity, and area of irradiation can be selected. Thus, in the transferring step, the material on the donor substrate is finally heated to allow its sublimation, and the material is thereby transferred onto the device substrate. Therefore, a film which is as uniform as in the case where the vapor deposition method is employed can be formed, and a high-performance device can hence be obtained.

In the production method of the present invention, an embodiment wherein the converting step (2) is included before the transferring step (3) (method I) and an embodiment wherein the converting step (2) is included at the same time with the transferring step (3) (method II) are preferred. In method I, the precursor material is applied on the donor substrate and then converted to the device material by the above-described method, followed by transfer to the device substrate. In method II, the precursor material is applied on the donor substrate, and the donor substrate and the device substrate are then superposed on each other to carry out transfer of the material to the device substrate. This transfer is carried out while converting the precursor material to a light emitting device material using the energy by heating or light irradiation during the transfer. In cases where the transfer is carried out with a laser light in the near infrared region, the laser light for the transfer and an ultraviolet or visible light for the conversion may be irradiated at the same time.

Among these, method I is preferred since it enables uniform conversion of the precursor material to the light emitting device material at a high conversion rate. In method I, a small amount of the precursor material may be remaining after the converting step. This is because the material is converted also in the subsequent transferring step.

Production of a device using the method of the present invention will now be described by way of examples of the method of production of an organic EL element, for which the method of the present invention is especially effective.

Fig. 1 is a cross-sectional view showing an example of a typical structure of an organic EL element (device substrate) 10, which is used as a display. On a support 11, an active matrix circuit is constituted, which active-matrix circuit is constituted by TFT 12 having an extraction electrode; a planarizing layer 13; and the like. The element portion has a first electrode 15/hole transporting layer 16/emitting layer 17/electron transporting layer 18/second electrode 19 formed on the active-matrix circuit. At the edge of the first electrode, an insulating layer 14 that prevents occurrence of short circuit at the electrode edge and defines the emitting region is formed. The constitution of the element is not restricted to this example. Examples thereof also include the followings. An element wherein a single emitting layer having both the hole transporting function and the electron transport function is formed between the first electrode and the second electrode. An element having a layered structure wherein the hole transporting layer has plural layers composed of a hole injection layer and a hole transporting layer, and the electron transporting layer has plural layers composed of an electron transporting layer and an electron injection layer. In cases where the emitting layer has an electron transport function, the electron transporting layer may be omitted. An element wherein the first electrode/electron transporting layer/emitting layer/hole transporting layer/second electrode are layered in that order.

Each of these layers may be composed of either a single layer or plural layers. Although not shown in the figure, after formation of the second electrode, formation of a protective layer, formation and sealing of a color filter, and/or the like may be carried out using known technologies.

In a color display, patterning of an emitting layer is at least necessary. As the light emitting device material precursor of the present invention, one which can be converted to a material used for the emitting layer is preferably used. Patterning of the insulating layer, first electrode, TFT and the like are often carried out by the known photolithography method, but the patterning may also be carried out using the light emitting device material precursor of the present invention by employing the method for producing the device. Further, also in cases where patterning of at least a single layer of the hole transporting layer, electron transporting layer and/or the like is necessary, the patterning may be carried out in the same manner. That is, using compounds of the present invention which can be converted to materials used for the TFT, materials used for the hole transporting layer, materials used for the electron transporting layer, and/or the like, the patterning can be carried out by the method for producing the device. Further, it is also possible to carry out patterning of only R and G among the emitting layers using the light emitting device material precursors according the method for producing the device, and form a layer thereon that works both as the emitting layer for B and as the electron transporting layers for R and G over the entire surface.

Examples of the method for preparing the organic EL element shown in Fig 1 include a method wherein the photolithography method is used until patterning of the first electrode 15 is carried out, and the photolithography method using a photosensitive polyimide precursor material is employed for patterning of the insulating layer 14, followed by whole-surface formation of the hole transporting layer 16 using a known technology by the vacuum deposition method. Using the hole transporting layer 16 as a bed layer, the emitting layers 17R, 17G and 17B, which give three colors, are patterned thereon by the method of the present invention. By forming the electron transporting layer 18 and the second electrode 19 on the whole surface of the resultant by a known technology such as the vacuum deposition method, the organic EL element can be completed.

The emitting layer may be composed of either a single layer or plural layers, and the light emitting device material on each layer may be either a single material or a mixture of plural materials. In view of the emission efficiency, color purity and durability, the emitting layer preferably has a single layer structure composed of a mixture of a host material and a dopant material. Therefore, the transfer material used for formation of the emitting layer is preferably a mixture of a host material and a dopant material. Since the ratio of the host material in the emitting layer is as high as 90 to 99% by weight, its ratio in the application liquid is also high. Therefore, whether or not the application liquid is successfully prepared depends on the solubility of the host material. From these reasons, as the light emitting device material precursor represented by General Formula (1), one which becomes a host material after conversion is preferably used. The emitting layer can be formed by applying a mixed solution of such a precursor material and a dopant material on a donor substrate and then drying the mixed solution, followed by the converting step and the transferring step.

Alternatively, a solution of a precursor material and a solution of a dopant material may be separately applied. Even in cases where the precursor material, host material and dopant material are not uniformly mixed on the donor substrate, it is sufficient if these are uniformly mixed when these are transferred to the organic EL element. Further, when the transfer is carried out, it is also possible to change the concentration of the dopant material in the emitting layer along the film-thickness direction using the difference in the evaporation temperature between the precursor material or host material and the dopant material.

Examples of the light emitting device material composition composed of the device material after conversion of the precursor material, and the dopant material, include those prepared by arbitrarily combining low-molecular-weight materials such as anthracene derivatives, tetracene derivatives, pyrene derivatives, quinolinol complexes including Tris(8-quinolinolato)aluminum (abbreviated as (Alq3)), various metal complexes including benzothiazolylphenol zinc complex, bisstyrylanthracene derivatives, tetraphenylbutadiene derivatives, coumarin derivatives, oxadiazole derivatives, benzoxazole derivatives, carbazole derivatives, distyrylbenzene derivatives, pyrrolopyridine derivatives, perinone derivatives, cyclopentadiene derivatives, oxadiazole derivatives, thiadiazolopyridine derivatives, rubrene, quinacridone derivatives, phenoxazone derivatives, perinone derivatives, perylene derivatives, coumarin derivatives, chrysene derivatives, pyrromethene derivatives, and iridium complex materials called phosphorescent materials; and/or macromolecular materials such as polyphenylenevinylene derivatives, polyparaphenylene derivatives and polythiophene derivatives. In particular, examples of materials excellent in the emission performance and suitable for the production method of the present invention include arbitrary combinations of anthracene derivatives, tetracene derivatives, pyrene derivatives, chrysene derivatives, pyrromethene derivatives, and various phosphorescent materials.

The hole transporting layer may be composed of either a single layer or plural layers, and each layer may be composed of either a single material or a mixture of plural materials. The layer called the hole injection layer is also included in the hole transporting layer. In view of the hole transportability (low driving voltage) and durability, an acceptor material for enhancement of the hole transportability may be blended in the hole transporting layer. Therefore, the transfer material which forms the hole transporting layer may be composed of either a single material or a mixture of plural materials.

Examples of the hole transport materials include low-molecular-weight materials such as aromatic amines represented by N,N'-di-[(1-naphthyl)-N,N'-diphenyl]-l,1'-biphenyl-4,4'-diamine (abbreviated as "α-NPD"), N,N,N',N'-tetrakis[(1,1'-biphenyl)-4-yl]-1,1'-diphenyl-4,4'-diamine, N,N'-bis[(9-carbazol-9-yl)phenyl]-N,N'-(diphenyl)-1,1'-biphenyl-4,4'-diamine and the like, N-isopropyl carbazol, pyrazoline derivatives, stilbene compounds, hydrazone compounds, oxadiazole derivatives, and heterocyclic compounds represented by phthalocyanine derivatives; and macromolecular materials such as polycarbonates, styrene derivatives, polyvinyl carbazoles and polysilanes having these low-molecular-weight compounds in their side chains. Examples of the acceptor material include low-molecular-weight materials such as 7,7,8,8-tetracyanoquinodimethane (abbreviated as "TCNQ"), and hexaazatriphenylene (abbreviated as "HAT") and its cyano group derivative hexacyanohexaazatriphenylene (abbreviated as "HAT-CN6"). Further examples of the hole transfer materials and the acceptor materials include metal oxides such as molybdenum oxide and silicon oxide thinly formed on the surface of the first electrode.

The electron transporting layer may be composed of either a single layer or plural layers, and each layer may be composed of either a single material or a mixture of plural materials. The layers called the hole inhibition layer and the electron injection layer are also included in the electron transporting layer. In view of the electron transportability (low driving voltage) and durability, a donor material for enhancement of the electron transportability may be blended in the electron transporting layer. The layer called the electron transporting layer is often regarded as the donor material. The transfer material which forms the electron transporting layer may be composed of either a single material or a mixture of plural materials.

Examples of the electron transport material include low-molecular-weight materials such as quinolinol complexes including Alq3 and 8-quinolinolato lithium (abbreviated as "Liq"), fused polycyclic aromatic derivatives including naphthalene and anthracene, styryl aromatic ring derivatives represented by 4,4'-bis(diphenylethenyl)biphenyl, quinone derivatives including anthraquinone and diphenoquinone, phosphorus oxide derivatives, benzoquinolinol complexes, hydroxyazole complexes, azomethine complexes, various metal complexes including tropolone metal complexes and flavonol metal complexes, and compounds having heteroaryl ring structures containing electron-accepting nitrogen; and macromolecular materials having these low-molecular-weight compounds in their side chains.

Examples of the donor material include various metal complexes containing alkali metals such as lithium and cesium and alkali earth metals such as magnesium and calcium, which constitute quinolinol complexes; and oxides and fluorides thereof such as lithium fluoride and cesium oxide. In order to extract emission from the emitting layer, at least one of the first electrode and the second electrode is preferably transparent. In the case of the bottom emission, wherein light is extracted from the first electrode, the first electrode is transparent, while in the case of the top emission, wherein light is extracted from the second electrode, the second electrode is transparent. When the transfer is performed, the so called reactive transfer, which includes, for example, reaction between the transfer material and oxygen, may be carried out. As the materials of the transparent electrode and the other electrode, known materials may be used as described in JP 11-214154 A, for example.

The organic EL element in the present invention is not restricted to the active-matrix type, wherein the second electrode is generally formed as a common electrode, and may also be the simple matrix type, which has a stripe electrode wherein the first electrode and the second electrode are crossed with each other, or the segment type, wherein the display portion is patterned such that predetermined information is displayed. Examples of uses thereof include televisions, personal computers, monitors, watches, thermometers, audio instruments, and display panels for automobiles.

The performance of the organic EL element produced by the method of the present invention is as high as that of an element prepared by the vapor deposition method in terms of the emission efficiency and the service life. Further, by the present invention, a large-scale organic EL element, whose production is difficult with the vapor deposition method, can be produced, and even in such a case, an element having an excellent emission efficiency and service life can be obtained.

### EXAMPLES

The present invention will now be described by way of Examples, but the present invention is not restricted by these Examples.

¹H-NMR (270 MHz) was carried out by using superconducting FT-NMR EX-270 (manufactured by JEOL Ltd.) and measuring with a deuterated chloroform solution.

### <<Example 1>> Synthesis of Compound (24)

Compound (24) was synthesized by the method shown in the reaction equation below. The particular process is described below.

[Chemical Formula 18]

### <Synthesis of 1-Ethynylpyrene (16)>

By the method described in "Journal of Physical Chemistry B", 2005, vol. 109, pp. 16628-16635, 1-ethynylpyrene (16) was synthesized. That is, 1-bromopyrene (14) (manufactured by Aldrich, 0.2 g, 0.71 mmol) and piperidine (40 mL) were placed in a two-necked flask, and a Dimroth condenser, a three-way cock and a septum were attached to the flask, followed by replacing the atmosphere in the flask with nitrogen. To the flask, 3-methyl-1-butyn-3-ol (12.9 g, 153 mmol), tetrakis(triphenylphosphine)palladium (0) (0.19 g, 0.16 mmol), triphenylphosphine (0.07 g, 0.29 mmol) and copper iodide (0.1 g, 0.53 mmol), and a solution of lithium bromide (0.3 g, 3.4 mmol) in tetrahydrofuran (abbreviated as "THF") (dry, 20 mL) were added in that order. The content of the flask was stirred while heating and stirring at 90°C for 3 hours. After completion of the reaction, the mixture was allowed to cool, and dichloromethane (70 mL) was added to the reaction liquid, followed by stirring the resulting mixture. The obtained solution was subjected to separation with dilute hydrochloric acid (5%, 300 mL), and the organic liquid layer was dried with sodium sulfate. The reaction product (15) was obtained by filtration/concentration and evaporation of the solvent, and dissolved in toluene without isolation, followed by adding potassium hydroxide (0.8 g, 14.3 mmol) thereto and heating and stirring the resulting mixture for 30 minutes. Potassium hydroxide (0.2 g, 3.6 mmol) was further added to the mixture, and the resulting mixture was heated and stirred for additional 30 minutes, followed by being allowed to cool to room temperature. The reaction liquid was filtered and then concentrated, followed by purification by silica gel chromatography (dichloromethane/hexane) to obtain 1-ethynylpyrene (16) (0.11 g, 70.8%). The measurement result by ¹H-NMR was as follows. (δ: ppm)
8.59 (d, J=8.9 Hz, 1H), 8.24-8.00 (m, 8H), 3.62 (s, 1H).

### <Synthesis of Alkynyl Sulfide (17)>

To a 200 mL two-necked flask, 1-ethynylpyrene (16) (1 g, 4.4 mmol) was placed, and a dropping funnel and a three-way cock were attached to the flask. The atmosphere in the flask was replaced with nitrogen, and tetrahydrofuran (dry, 10 mL) was added thereto, followed by cooling the resulting mixture to -78°C with stirring. N-butyllithium (1.6 M solution in hexane, 3.0 mL, 4.8 mmol) was added dropwise thereto. After completion of the dropwise addition, stirring was continued at -78°C for 30 minutes. A solution of p-tolyl disulfide (1.1 g, 4.5 mmol) in tetrahydrofuran (dry, 10 mL) was added dropwise to the mixture. The reaction solution was allowed to warm to room temperature, and stirred for additional 1.5 hours. The reaction liquid was cooled to -40°C, and a solution of p-nitrobenzylbromide (1.1 g, 5.1 mmol) in tetrahydrofuran (dry, 10 mL) was added dropwise thereto. The reaction solution was allowed to warm to room temperature again, and stirred for additional 1.5 hours.

An aqueous ammonium chloride solution was added to the reaction solution, and the resulting mixture was subjected to separation by addition of diethylether. The aqueous layer was extracted with diethylether, and the resultant was combined with the organic liquid layer obtained by the separation, followed by drying the mixture with magnesium sulfate. The reaction product was filtered and concentrated, and purified by silica gel chromatography (ethyl acetate/hexane) to obtain alkynyl sulfide (17) (1.4 g, 85.7%). The measurement result by ¹H-NMR was as follows. (δ: ppm)
8.58 (d, J=9.2 Hz, 1H), 8.24-8.03 (m, 8H), 7.53 (d, J=8.4 Hz, 2H), 7.22 (d, J=8.4 Hz, 2H), 2.37 (s, 3H).

### <Synthesis of Tosylethynyl Derivative (18)>

Alkynyl sulfide (17) (1.4 g, 4.1 mmol) was placed in a flask, and dissolved in dry dichloromethane (dry, 15 mL). While cooling the reaction solution on ice, a suspension of m-chloroperbenzoic acid (mCPBA) (65%, 2.4 g, 9.1 mmol) in dichloromethane (dry, 40 mL) was added dropwise thereto. The reaction liquid was allowed to warm to room temperature, and stirring of the reaction liquid was continued for 1 hour. After completion of the reaction, the reaction solution was filtered, and the resulting filtrate was subjected to 3 times of separation with an aqueous sodium carbonate solution. The organic liquid layer was dried with magnesium sulfate, and then filtered and concentrated. The obtained reaction product was purified by silica gel chromatography (dichloromethane/hexane), to obtain a tosylethynyl derivative (18). Yield: 1.1 g (70.0%).
The measurement result by ¹H-NMR was as follows. (δ: ppm))
8.37-8.04 (m, 11H), 7.44 (d, J=8.6 Hz, 2H), 2.49 (s, 3H).

### <Synthesis of Intermediate (20)>

An acetonide derivative (19) of a 3,5-cyclohexadiene-1,2-diol derivative was synthesized as follows. In 2,2-dimethoxypropane, 3-bromo-3,5-cyclohexadiene-1,2-diol (manufactured by Aldrich, 0.5 g, 2.6 mmol) was stirred for 30 minutes in the presence of p-toluenesulfonic acid monohydrate (0.01 g, 0.05 mmol). The reaction solution was passed through a silica gel short column to obtain a solution containing an acetonide derivative (19), which was then concentrated to be used in the subsequent reaction as it is.

The concentrate containing the acetonide derivative (19) and the tosylethynyl derivative (18) (0.5 g, 1.3 mmol) were heated and stirred in toluene at 60°C for 72 hours. After being allowed to cool to room temperature, the reaction liquid was passed through a silica gel short column, and the compound (20) recovered was used as it is in the subsequent reaction.

### <Synthesis of Intermediate (21)>

The above-described compound (20) was placed in a 100 mL two-necked flask, and the atmosphere in the flask replaced with argon. To this flask, samarium iodide (0.1 M solution in tetrahydrofuran, 100 mL, 10 mmol) was added. The obtained solution was cooled to -20°C, and hexamethylphosphoric triamide (10 mL) was added dropwise thereto. The resulting mixture was stirred for 30 minutes while keeping the temperature at -20°C, and the reaction liquid was then allowed to warm to room temperature. An aqueous ammonium chloride solution was added to the reaction liquid, and the reaction liquid was then concentrated. The concentrate containing the thus obtained reaction product (21) was used as it is in the subsequent reaction.

### <Synthesis of Intermediate (22)>

Toluene (20 mL) was added to the concentrate containing the reaction product (21), and separation was attained with an aqueous sodium thiosulfate solution, followed by drying the organic liquid layer with sodium sulfate. The solution was filtered and concentrated, and methanol and pyridinium p-toluenesulfonate (2.17 g, 0.87 mmol) were added to the resulting solution, followed by heating and stirring the resulting mixture at about 80°C. The heating and stirring were continued for 24 hours while adding methanol and toluene as required. After completion of the reaction, the solution was allowed to cool to room temperature, and the solvent was evaporated under reduced pressure. The obtained crude was passed through a silica gel short column to obtain a reaction product (22), which was used as it is in the subsequent reaction.

### <Synthesis of Compound (23)>

Dimethylsulfoxide (dry, 10 mL) was dissolved in dichloromethane (dry, 10 mL), and the resulting solution was cooled to -78°C. Trifluoroacetic anhydride (TFAA) (18.7 mL) was added dropwise to the solution, and the resulting mixture was stirred at -78°C for 15 minutes. To this mixture, a solution of the above-described reaction product (22) in dimethylsulfoxide (DMSO) (dry, 10 mL) was added dropwise slowly, and the resulting mixture was stirred at -78°C for 90 minutes. Subsequently, triethylamine (20 mL) was added dropwise, and the resulting mixture was further stirred at -78°C for 90 minutes, followed by allowing the reaction liquid to warm to room temperature. After completion of the reaction, dichloromethane was added thereto, and the resulting mixture was stirred, followed by washing the organic liquid layer with water. After separation, the organic liquid layer was dried with sodium sulfate, and the filtrate obtained by filtration was concentrated to dryness. By purifying the obtained solids by silica gel chromatography, (23) was obtained. Yield:0.08g. The measurement result by ¹H-NMR was as follows. (δ: ppm)
8.40-8.12 (m, 9H), 6.3 (s, br, 1H), 6.13-6.10 (m, 2H), 4.40 (s, 1H), 4.21 (s, 1H).

This compound (24) could be prepared into 3% by weight solution in any of the following solvents: chloroform, toluene and tetralin.

### <<Example 2>> Conversion to Light Emitting Device Material by Light Irradiation

A solution of compound (23) in toluene (3% by weight) was left to stand under white indoor light for 12 hours, and the component in the solution was then analyzed by HPLC. As a result, only a peak which could be identified as 1-phenylpyrene, which is a light emitting device material, was found. Thus, it was confirmed that the compound (23) can be converted to a light emitting device material by skeletal transformation by light irradiation.

### <<Example 3>> Synthesis of Compound (36)

Compound (36) was synthesized by the method shown in the reaction equation below.

[Chemical Formula 19]

### <Synthesis of 1-(4-Chlorophenyl)pyrene (26)>

In a three-necked flask, 1-bromopyrene (20 g, 71 mmol), 4-chlorophenylboronic acid (12.2 g, 78 mmol) and palladium acetate (8 mg, 0.03 mmol) were placed, and a Dimroth condenser, a three-way cock and a septum were attached to the flask. The atmosphere in the flask was replaced with nitrogen, and 1,2-dimethoxyethane (360 mL) which had been preliminarily subjected to nitrogen bubbling was added to the flask, followed by stirring the resulting mixture. To this suspension, 2 M aqueous sodium carbonate solution (80 mL) was added, and the atmosphere was replaced with nitrogen, followed by heating the mixture to reflux for 6.5 hours. After completion of the reaction, the reaction liquid was allowed to cool to room temperature, and water (300 mL) was added thereto, followed by vigorous stirring of the liquid. The precipitated solids are filtered, and the obtained solids were further washed with water (300 mL). The solids were filtered and washed with methanol (100 mL), and then dried sufficiently. The obtained crude was purified through a silica gel short column (developing solvent: toluene), to obtain 1-(4-chlorophenyl)pyrene (26) as yellowish white solids (Yield: 21 g; Percent Yield: 94%). This compound was confirmed to have a purity (area percentage) of not less than 95% in HPLC, and then used as it is in the subsequent reaction.

### <Bromination of 1-(4-Chlorophenyl)pyrene>

The intermediate (26) (20 g, 64 mmol) and N-bromosuccinimide (11.4 g, 64 mmol) were placed in a 1 L three-necked flask, and a three-way cock, a Dimroth condenser, and a septum were attached to the flask. The atmosphere in the flask was replaced with nitrogen, and 1,2-dimethoxyethane (400 mL) which had been preliminarily subjected to nitrogen bubbling was added to the flask, followed by heating and stirring the resulting mixture for 7 hours. After completion of the reaction, the reaction liquid was allowed to cool to room temperature, and the precipitated solids were separated by filtration. The obtained solids were washed with water (200 mL) and then with methanol (100 mL), and dried sufficiently in a vacuum drier, to obtain bromo-(4-chlorophenyl)pyrene (27) as white solids (Yield: 24.2 g; Percent Yield: 92%). This compound was a mixture of isomers, but used in the subsequent reaction without separation/purification.

### <Synthesis of Intermediate (28)>

The intermediate (27) (15 g, 38 mmol) and piperidine (100 mL) were placed in a two-necked flask, and a Dimroth condenser, a three-way cock and a septum were attached to the flask, followed by replacing the atmosphere in the flask with nitrogen. To the flask, 3-methyl-1-butyn-3-ol (80 mL), bis(triphenylphosphine)palladium (II) dichloride (0.38 g, 0.54 mmol), triphenylphosphine (0.55 g, 2.1 mmol), copper iodide (1.87 g, 9.84 mmol), and a solution of lithium bromide (4.2 g, 48 mmol) in tetrahydrofuran (THF) (dry, 180 mL) were added in that order. The content of the flask was heated and stirred at 90°C for 12 hours with stirring. After completion of the reaction, the reaction liquid was allowed to cool, and toluene (500 mL) was added thereto, followed by stirring the resulting mixture. The obtained solution was subjected to separation with dilute hydrochloric acid (10%, 350 mL), water and saturated brine, and dried with sodium sulfate. The crude was purified by silica gel chromatography (toluene, followed by toluene/dichloromethane=1:1), to obtain the intermediate of interest (28) as yellow solids (Yield: 5.4 g; Percent Yield: 36%).
The intermediate (28) was separated into isomers at this stage. ¹H-NMR (δ: ppm) 8.55 (d, 1H, J=9.5 Hz), 8.27-7.94 (m, 7H), 7.55 (s, br, 4H), 2.20 (s, 1H), 1.81 (s, 6H).

### <Synthesis of Intermediate (29)>

The intermediate (28) (2.4 g, 6.1 mmol) was dissolved in toluene, and potassium hydroxide (0.8 g, 171 mmol) was added thereto, followed by heating and stirring the resulting mixture for 30 minutes. Potassium hydroxide (2.4 g, 43 mmol) was further added to the mixture, and the resulting mixture was heated and stirred for additional 30 minutes, followed by allowing the mixture to cool to room temperature. The reaction liquid was filtered through Celite and then concentrated, followed by purification by silica gel chromatography (toluene), to obtain a 1-ethynyl pyrene derivative (29) (Yield: 1.6 g, Percent Yield: 80%). The result of ¹H-NMR was as follows. (δ: ppm)
8.55 (d, 1H, J=10.0 Hz), 8.28-7.96 (m, 7H), 7.55 (s, br, 4H), 3.61 (s, 1H).

### <Synthesis of Intermediate (30)>

In a 200 mL two-necked flask, the 1-ethynylpyrene derivative (29) (0.75 g, 0.23 mmol) was placed, and a dropping funnel and a three-way cock were attached to the flask. The atmosphere in the flask was replaced with nitrogen, and tetrahydrofuran (dry, 8 mL) was added thereto, followed by cooling the resulting mixture to -78°C with stirring. N-butyllithium (1.6 M solution in hexane, 2.0 mL, 3.2 mmol) was added dropwise thereto. After completion of the dropwise addition, stirring was continued at -78°C for 30 minutes. A solution of p-tolyl disulfide (0.66 g, 2.7 mmol) in tetrahydrofuran (dry, 8 mL) was added dropwise to the mixture.
The reaction solution was allowed to warm to room temperature, and stirred for additional 1.5 hours. The reaction liquid was cooled to -40°C, and a solution of p-nitrobenzylbromide (0.48 g, 2.2 mmol) in tetrahydrofuran (dry, 8 mL) was added dropwise thereto. The reaction solution was allowed to warm to room temperature again, and stirred for additional 1.5 hours.

An aqueous ammonium chloride solution was added to the reaction solution, and the resulting mixture was subjected to separation by addition of diethylether. The aqueous layer was extracted with diethylether, and the resultant was combined with the organic liquid layer, followed by drying the mixture with magnesium sulfate. The reaction product was filtered and concentrated, and purified by silica gel chromatography (dichloromethane/hexane) to obtain an intermediate (30) (Yield: 0.75g, 73.5%). The measurement result by ¹H-NMR was as follows. (δ: ppm) 8.53 (d, 1H, J=8.9Hz), 8.20-7.94 (m, 7H), 7.54-7.51 (m, 6H), 7.25-7.21 (m, 2H), 2.35 (s, 3H).

### <Synthesis of Intermediate (31)>

The intermediate (30) (0.75 g, 1.6 mmol) was placed in a flask, and dissolved in dry dichloromethane (dry, 25 mL). While cooling the reaction solution on ice, a solution of m-chloroperbenzoic acid (mCPBA) (65%, 1.1 g, 4.1 mmol) in dichloromethane (dry, 20 mL) was added dropwise thereto. The reaction liquid was allowed to warm to room temperature, and stirring of the reaction liquid was continued for 1 hour. After completion of the reaction, the reaction solution was subjected to separation with an aqueous sodium carbonate solution. The organic liquid layer was dried with magnesium sulfate, and then filtered and concentrated. The obtained reaction product was purified by silica gel chromatography (dichloromethane), to obtain an intermediate (31) (Yield: 0.72 g; Percent Yield: 90.0%). The measurement result by ¹H-NMR was as follows (δ: ppm).
8.35-7.97 (m, 8H), 7.54 (s, br, 4H), 7.45 (d, 2H, J=5.4 Hz), 7.11 (d, 2H, J=5.2 Hz), 2.37 (s, 3H).

### <Synthesis of Intermediate (32)>

The concentrate containing the acetonide body (19), and the intermediate (31) (0.72 g, 1.5 mmol) were heated and stirred in toluene at 60°C for 20 hours. After being allowed to cool to room temperature, the reaction liquid was concentrated to dryness, and dissolved in dichloromethane, followed by purification by silica gel chromatography (dichloromethane), to obtain an intermediate (32) (Yield: 0.5 g; Percent Yield: 47.2%). This compound was confirmed by HPLC to be composed of a single component, and used as it is in the subsequent reaction.

### <Synthesis of Intermediate (33)>

The intermediate (32) (0.24 g, 0.33 mmol), 4-dibenzofuranboronic acid (0.11 g, 0.52 mmol), allyl[1,3-bis(2,6-diisopropylphenyl)imidazol-2-ylidene]palladium chloride (0.017 g, 0.03 mmol) and sodium t-butoxide (0.11 g, 1.0 mmol) were placed in a 100 mL two-necked flask, and a Dimroth condenser, a three-way cock and a septum were attached to the flask. The atmosphere in the flask was replaced with nitrogen, and tetrahydrofuran (dry, 3 mL) and methanol (dry, 3 mL) were added thereto, followed by heating the resulting mixture at 55°C for 9 hours. After completion of the reaction, the mixture was allowed to cool to room temperature, and the solvent was evaporated under reduced pressure, to obtain a crude. The crude was purified by silica gel chromatography (dichloromethane), to obtain an intermediate (33) (Yield: 0.16 g; Percent Yield: 57.7%). This compound was confirmed by HPLC to be composed of a single component, and used as it is in the subsequent reaction.

### <Synthesis of Intermediate (34)>

The compound (33) (0.076 g, 0.09 mmol) was placed in a 100 mL two-necked flask, and the atmosphere in the flask was replaced with argon. Samarium iodide (0.1 M solution in tetrahydrofuran, 4.0 mL, 0.4 mmol) was added thereto. The obtained solution was cooled to -20°C, and hexamethylphosphoric triamide (0.4 mL) was added dropwise thereto. The resulting mixture was stirred for 30 minutes while keeping the temperature at -20°C, and the reaction liquid was then allowed to warm to room temperature. An aqueous ammonium chloride solution was added to the reaction liquid, and the reaction liquid was then concentrated. Dilute hydrochloric acid (10%, 20 mL) was added to the concentrated reaction liquid, and the resulting mixture was filtered, followed by dissolving the obtained solids in ethyl acetate. This solution was subjected to separation with saturated brine, and the organic liquid layer was dried with magnesium sulfate and filtered, followed by evaporating the filtrate under reduced pressure. The obtained crude was purified by silica gel chromatography (dichloromethane), to obtain an intermediate (34) as yellowish solids (Yield: 40 mg, Percent Yield: 71.6%). The measurement result by ¹H-NMR was as follows. (δ: ppm)
8.31-7.48 (m, 19H), 6.72-6.48 (m, 3H), 4.69-4.57 (m, 2H), 4.28 (s, br, 2H), 4.13 (s, br, 2H), 1.43 (s, 3H), 1.31 (s, 3H).

### <Synthesis of Intermediate (35)>

The intermediate (34) (60 mg, 0.09 mmol) was placed in a recovery flask, and dissolved in toluene (15 mL) and methanol (30 mL). Pyridinium p-toluenesulfonate (0.15 mg, 0.06 mmol) was added to the resulting solution, followed by heating and stirring the resulting mixture at about 80°C. The heating and stirring were continued for 24 hours while adding methanol and toluene as required. After completion of the reaction, the solution was allowed to cool to room temperature and the solvent was evaporated under reduced pressure. The obtained crude was purified by silica gel chromatography (dichloromethane), to obtain an intermediate (35) as white solids. (Yield: 38 mg; Percent Yield: 73.1%)
The measurement result by ¹H-NMR was as follows. (δ: ppm)
8.31-7.17 (m, 19H), 6.81-6.42 (m, 3H), 4.28-4.07 (m, 4H), 2.61 (s, br, 2H).

### <Synthesis of Compound (36)>

Dimethylsulfoxide (dry, 1 mL) was dissolved in dichloromethane (dry, 10 mL), and cooled to -78°C. Trifluoroacetic anhydride (TFAA) (2 mL) was added dropwise to the solution, and the resulting mixture was stirred at -78°C for 15 minutes. To this mixture, a solution of the intermediate (35) in dimethylsulfoxide (DMSO) (dry, 5 mL) was added dropwise slowly, and the resulting mixture was stirred at -78°C for 90 minutes. Subsequently, N,N-diisopropylethylamine (5 mL) was added dropwise thereto, and the resulting mixture was further stirred at -78°C for 90 minutes, followed by allowing the reaction liquid to warm to room temperature. After completion of the reaction, dichloromethane was added to the liquid, and the resulting mixture was stirred, followed by washing the organic liquid layer with water. After separation, the organic liquid layer was dried with sodium sulfate, and the filtrate obtained by filtration was concentrated to dryness. By purifying the obtained solids by silica gel chromatography, (36) was obtained (Yield: 20 mg; Percent Yield: 53.1 %). The measurement result by ¹H-NMR was as follows. (δ: ppm)
8.39-7.38 (m, 19H), 7.01-6.79 (m, 3H), 4.71 (s, br, 1H), 4.52 (s, br, 1H).
This compound (36) could be prepared into 2% by weight solution in any of the following solvents: chloroform, toluene and tetralin.

### «Example 4» Conversion to Light Emitting Device Material by Light Irradiation

A glass substrate was spin-coated with a solution of the compound (36) in toluene (1% by weight) to prepare a thin film, and light of a UV light emitting diode (peak wavelength, 380 nm; half bandwidth, 20 nm) was irradiated to the thin film at room temperature under nitrogen atmosphere for 1 hour. The organic substance on the glass substrate was extracted with dichloromethane, and the component of the resulting solution was analyzed by HPLC. As a result, only a peak which could be identified as the compound (37), which is a light emitting device material, was found. Thus, it was confirmed that the compound (36) can be converted to a light emitting device material by skeletal transformation by light irradiation.

[Chemical Formula 20]

### «Example 5» Conversion to Light Emitting Device Material by Light Irradiation

Light irradiation was carried out in the same manner as in Example 4 except that the light of the UV light emitting diode (peak wavelength, 380 nm; half bandwidth, 20 nm) was changed to light of a blue light emitting diode (peak wavelength, 460 nm; half bandwidth, 20 nm). As a result, the compound (36) and the compound (37) were observed at ratios of 3% and 97%, respectively.

### «Example 6» Preparation of Organic EL Element

A donor substrate was prepared as follows. As a support, an alkali-free glass substrate was used, and after washing/UV ozone treatment thereof, a tantalum film having a thickness of 0.4 µm was formed as a photothermal conversion layer by the sputtering method over the whole surface. Subsequently, the photothermal conversion layer was subjected to UV ozone treatment. On this layer, a positive-type polyimide photosensitive coating agent (manufactured by TORAY INDUSTRIES, INC., DL-1000) was applied by spin coating after concentration adjustment, and prebaking and pattern exposure by UV were performed, followed by dissolving/removing of the exposed portion by a developer (manufactured by TORAY INDUSTRIES, INC., ELM-D). The thus patterned polyimide precursor film was baked at 300°C for 10 minutes, to form a polyimide compartment pattern. The height of this compartment pattern was 7 µm, and its cross section was in a forward tapered shape. In the compartment pattern, apertural areas each having a width of 80 µm and a length of 280 µm for exposure of the photothermal conversion layer were arranged at pitches of 100 µm and 300 µm. On this substrate, a solution containing 1 wt% compound (36) and, as a dopant, C545T in an amount of 0.8 wt% with respect to the compound (36), in chloroform was applied by spin coating, and the solution was then dried. To the obtained thin film, light of a light emitting diode (peak wavelength, 380 nm; half bandwidth, 20 nm) was irradiated under nitrogen atmosphere for 1 hour, to convert the compound (36) to the compound (37). As a result, a layer having an average thickness of 25 nm was formed in the compartment pattern (apertural areas), which layer was composed of the compound 37 and C545T (manufactured by Lumitec). The content of the compound (36) in the thin film prepared under these conditions was analyzed by HPLC, and the content was estimated to be 0.1%.

A device substrate was prepared as follows. An alkali-free glass substrate on which ITO transparent conductive coating having a thickness of 140 nm was deposited (manufactured by Geomatec Co., Ltd., sputter-deposited product) was cut into a piece having a size of 38×46 mm, and the ITO was etched into a desired shape by photolithography. Thereafter, a polyimide precursor film patterned in the same manner as the donor substrate was baked at 300°C for 10 minutes, to form a polyimide insulating layer. The height of this insulating layer was 1.8 µm, and its cross section was in a forward tapered shape. In the pattern of the insulating layer, apertural areas each having a width of 70 µm and a length of 270 µm were arranged at pitches of 100 µm and 300 µm. This substrate was subjected to UV/ozone treatment, and placed in a vacuum deposition apparatus, followed by evacuation until the degree of vacuum in the apparatus becomes not more than 3×10⁻⁴ Pa. By resistance heating, the compound having the structure shown below (HIL1), and NPD as a hole transporting layer, were layered with thicknesses of 50 nm and 10 nm, respectively, over the whole emitting region by vapor deposition.

Thereafter, the position of the compartment pattern of the donor substrate and the position of the insulating layer of the device substrate were aligned and made to face each other, and the substrates were kept in a vacuum of not more than 3×10⁻⁴ Pa, followed by removing these into the air. The transfer space partitioned by the insulating layer and the compartment pattern was kept in vacuum. For the transfer, light having a center wavelength of 940 nm whose irradiation shape was formed into a rectangle of 340 µm×50 µm (light source: semiconductor laser diode) was used. The light was irradiated from the glass substrate side of the donor substrate such that the longitudinal direction of the compartment pattern and the insulating layer corresponded to the longitudinal direction of the light, and scanning was performed in the longitudinal direction such that the transfer material and the compartment pattern were heated at the same time, thereby transferring the coevaporated film as the transfer material onto the hole transporting layer, which is the bed layer of the device substrate. The light intensity was adjusted within the range of 140 to 180 W/mm², and the scanning rate was 0.6 m/s. The scan was repeatedly carried out such that the transfer is performed over the whole emitting region while allowing the light to overlap in the transverse direction at a pitch of about 300 µm.
The device substrate after the transfer was placed in the vacuum deposition apparatus again, followed by evacuation until the degree of vacuum in the apparatus becomes not more than 3×10⁻⁴ Pa. By resistance heating, the compound shown in E-1 below was vapor-deposited, as the electron transporting layer, over the whole emitting region to a thickness of 25 nm. Subsequently, lithium fluoride as the donor material (electron injection layer) and aluminum as the second electrode were vapor-deposited to thicknesses of 0.5 nm and 65 nm, respectively, to prepare an organic EL element having an emitting region of 5 mm×5 mm. As a result, obvious green emission was confirmed.

[Chemical Formula 21]

### «Examples 7 to 13»

Organic EL elements were prepared in the same manner as in Example 6 except that the irradiated light and the irradiation time were changed as shown in Table 1. The contents of the compound (36) in the thin films prepared under these conditions were analyzed by HPLC. The results are shown in Table 1.

### «Reference Example 1>>

An organic EL element was prepared in the same manner as in Example 6 except that the light irradiation was not performed. The content of the compound (36) in the thin film prepared under these conditions was analyzed by HPLC. Emission as observed in Example 6 could not be further observed in this experiment.

### «Examples 14 to 20»

Organic EL elements were prepared in the same manner as in Example 6 except that BD-1 shown below was used instead of C545T used in Example 6 as a dopant, and the irradiated light and the irradiation time were changed as shown in Table 1. As a result, obvious blue emission was confirmed.

[Chemical Formula 22]

### «Reference Example 2»

An organic EL element was prepared in the same manner as in Example 14 except that the light irradiation was not performed. The content of the compound (36) in the thin film prepared under these conditions was analyzed by HPLC. Emission as observed in Example 6 could not be further observed in this experiment.

### «Comparative Example 1>>

In Example 6, a solution containing 1 wt% compound (36) and, as a dopant, C545T in an amount of 0.8 wt% with respect to the compound (36), in chloroform was used when the donor substrate was prepared, but, in the present Comparative Example, a suspension containing 1 wt% compound (37) and, as a dopant, C545T in an amount of 0.8 wt% with respect to the compound (36), in chloroform was used instead. An organic EL element was prepared in the same manner as in Example 6 except for the above alteration. Since, at this time, the compound (37) was in the form of a suspension wherein the compound was not dissolved completely, a uniform film could not be prepared on the donor substrate. Accordingly, an excellent film could not be transferred onto the device substrate, and emission could not be confirmed.

### «Comparative Example 2»

An organic EL element was prepared in the same manner as in Comparative Example 2 except that BD-1 shown below was used as a dopant instead of using C545T. Also at this time, as in Comparative Example 1, a uniform film could not be prepared on the donor substrate, so that an excellent film could not be transferred onto the device substrate, and EL emission could not be confirmed.

### «Initial Emission Efficiency, Luminance Half-life>>

After sealing the organic EL elements prepared in Examples 6 to 21 and Comparative Examples 1 to 2, a constant current of 2.5 mA/cm² was applied thereto. Measurement was carried out by defining the luminance immediately after initiating the application as the initial luminance, the value calculated by dividing the initial luminance by the current density as the initial emission efficiency, and the time required for the luminance to decrease to a half of the initial luminance by keeping application of the constant current as the luminance half-life. The relative ratios of the measurement values for Examples 7 to 13 and Comparative Example 1 calculated taking the measurement value for Example 6 as 1.0 are summarized in Table 1 as the relative initial emission efficiency and the relative luminance half-life. Further, the relative ratios of the measurement values for Examples 15 to 21 and Comparative Example 2 calculated taking the measurement value for Example 14 as 1.0 are summarized in Table 1 as the relative initial emission efficiency and the relative luminance half-life.

[Table 1]

The effect of the light emitting device material precursor of the present invention could be confirmed by its skeletal transformation by light irradiation after coating film formation, in preparation of a thin film of a compound with which coating film formation had been conventionally difficult. Accordingly, the efficiency and the life of the organic light emitting device were improved.

### DESCRIPTION OF SYMBOLS

- 10.: Organic EL element (device substrate)
- 11.: Support
- 12.: TFT
- 13.: Planarizing layer
- 14.: Insulating layer
- 15.: First electrode
- 16.: Hole transporting layer
- 17.: Emitting layer
- 18.: Electron transporting layer
- 19.: Second electrode

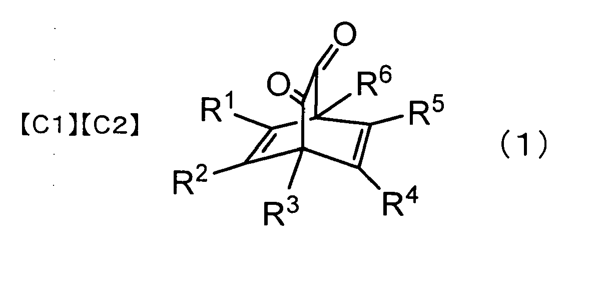

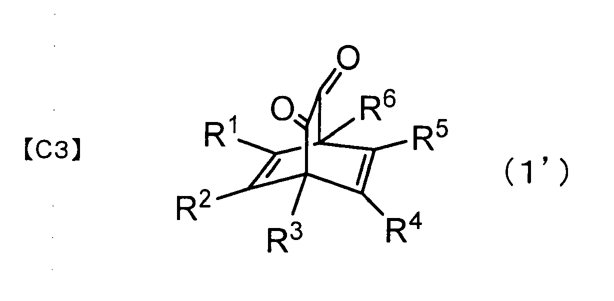

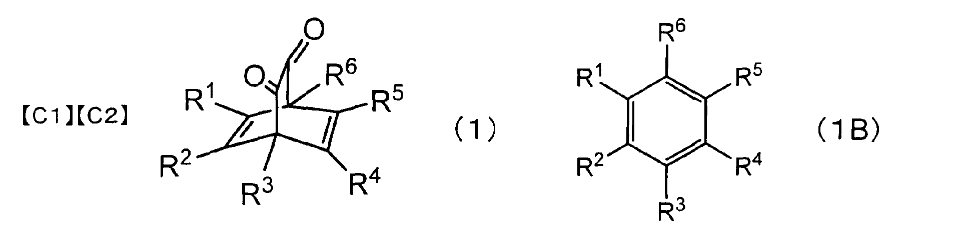

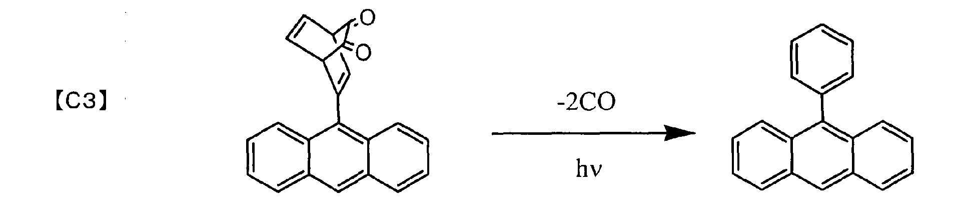

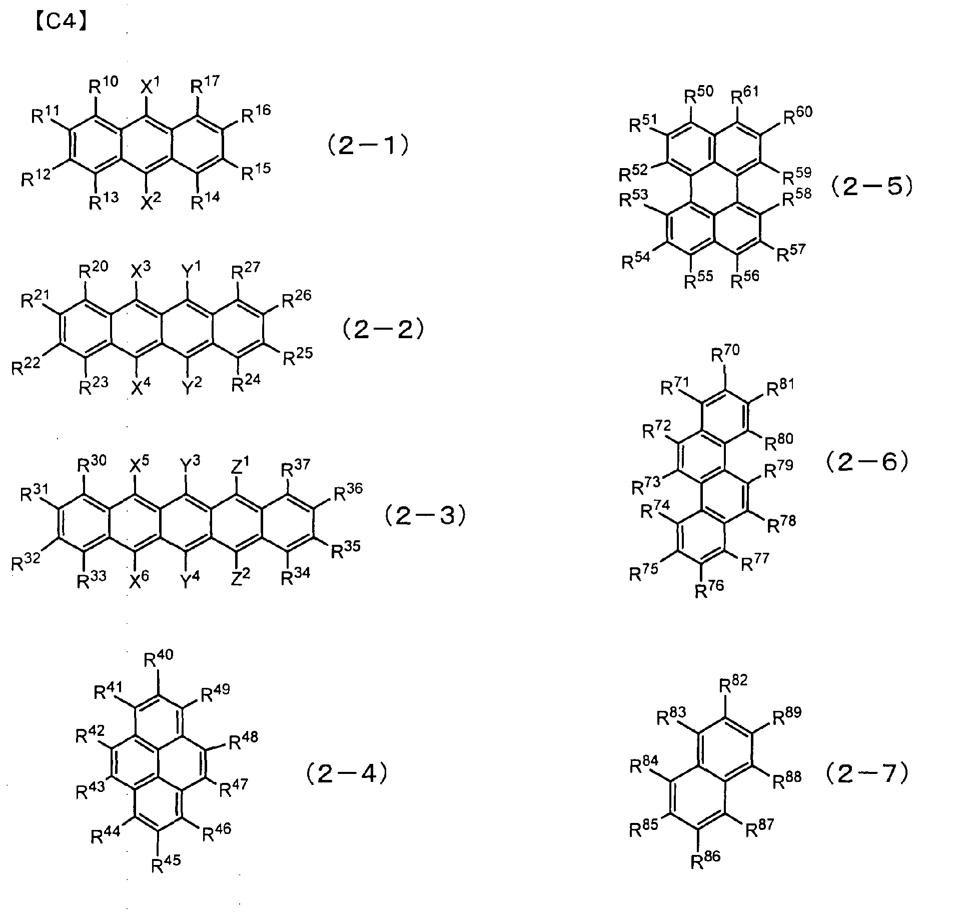

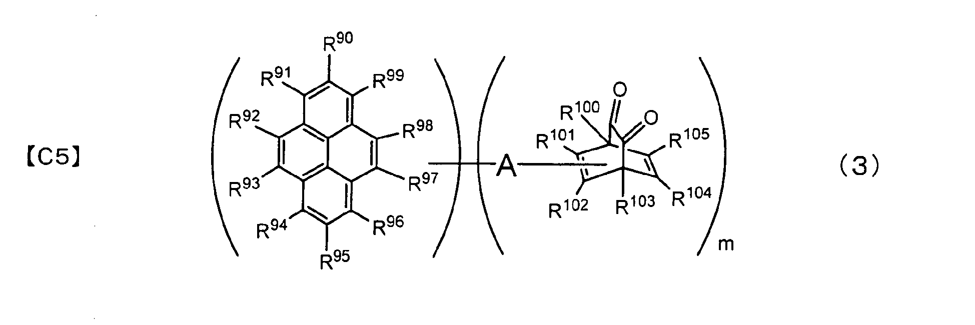

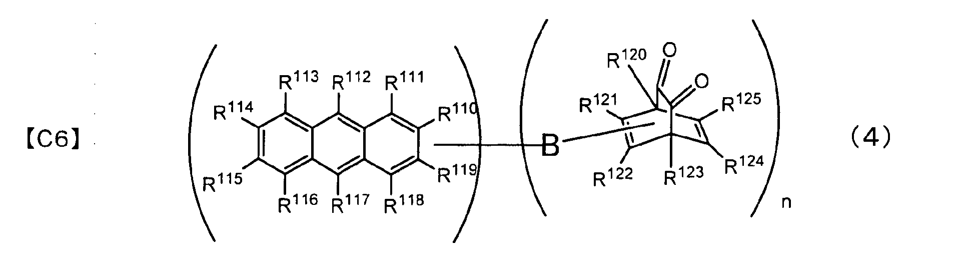

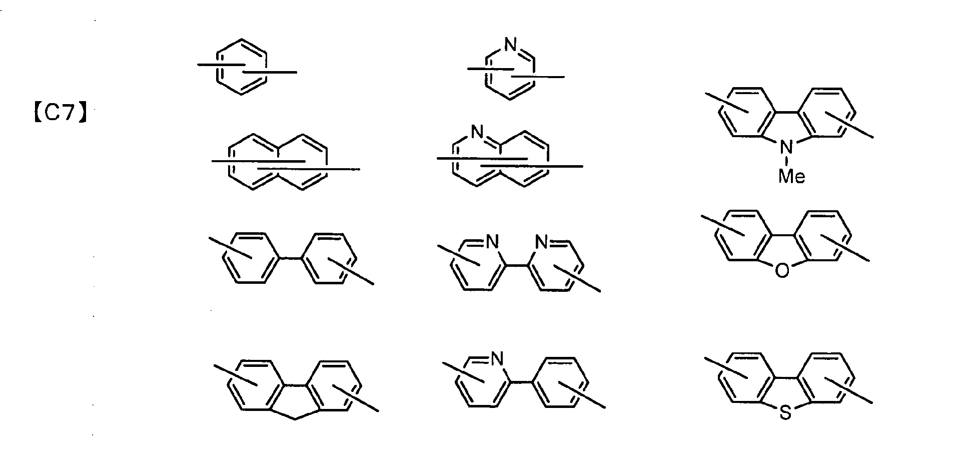

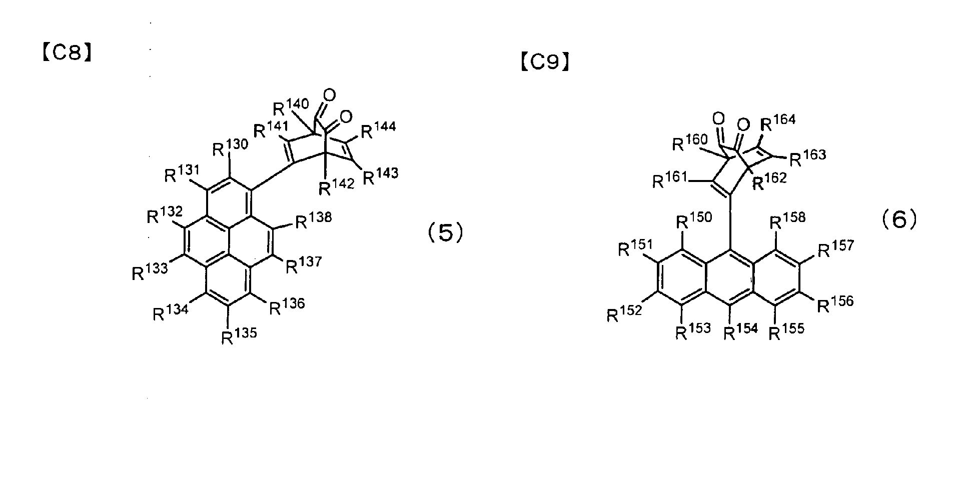

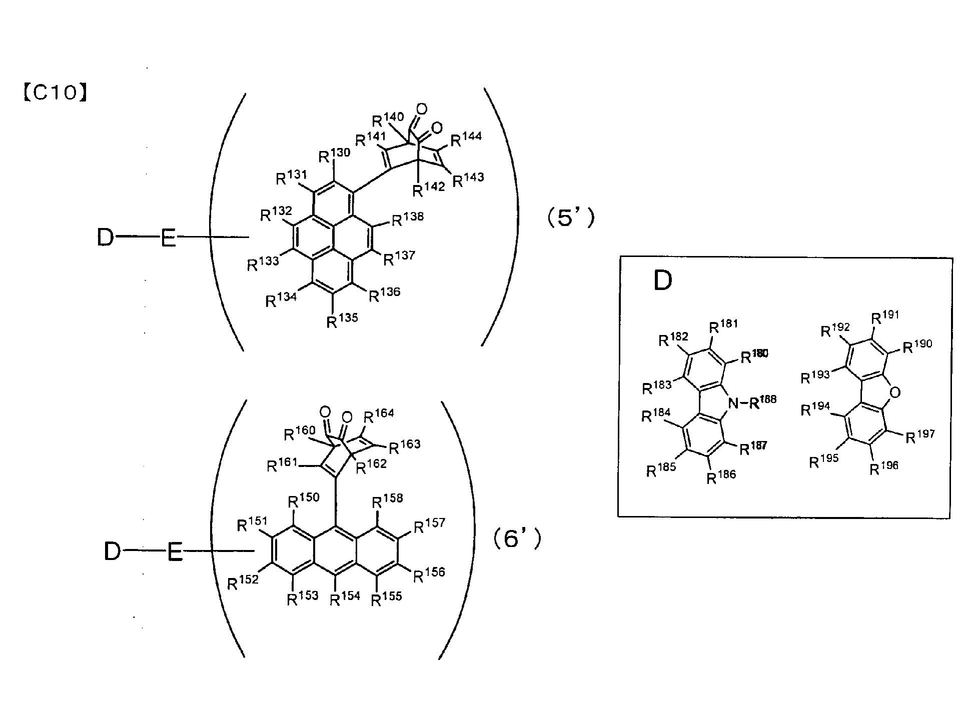

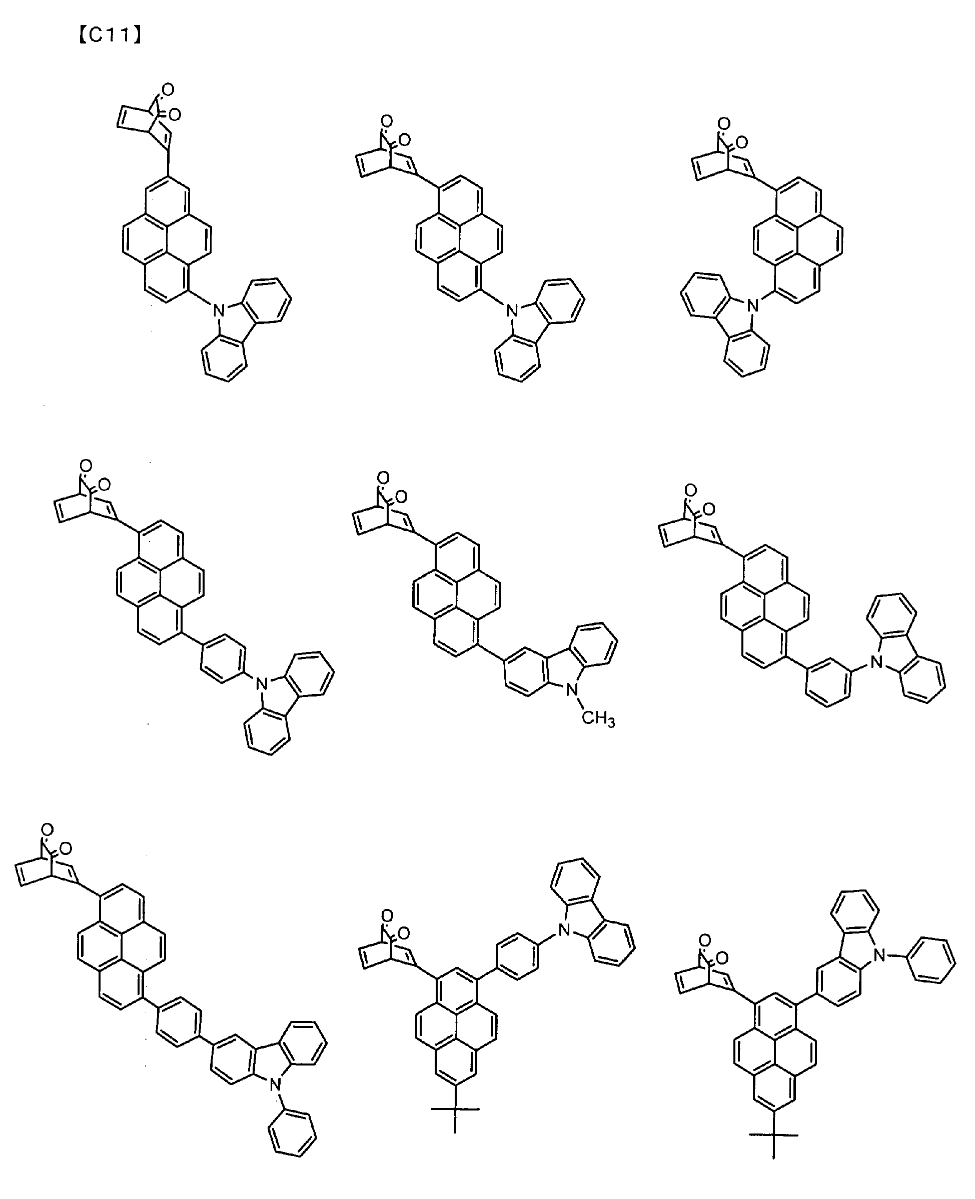

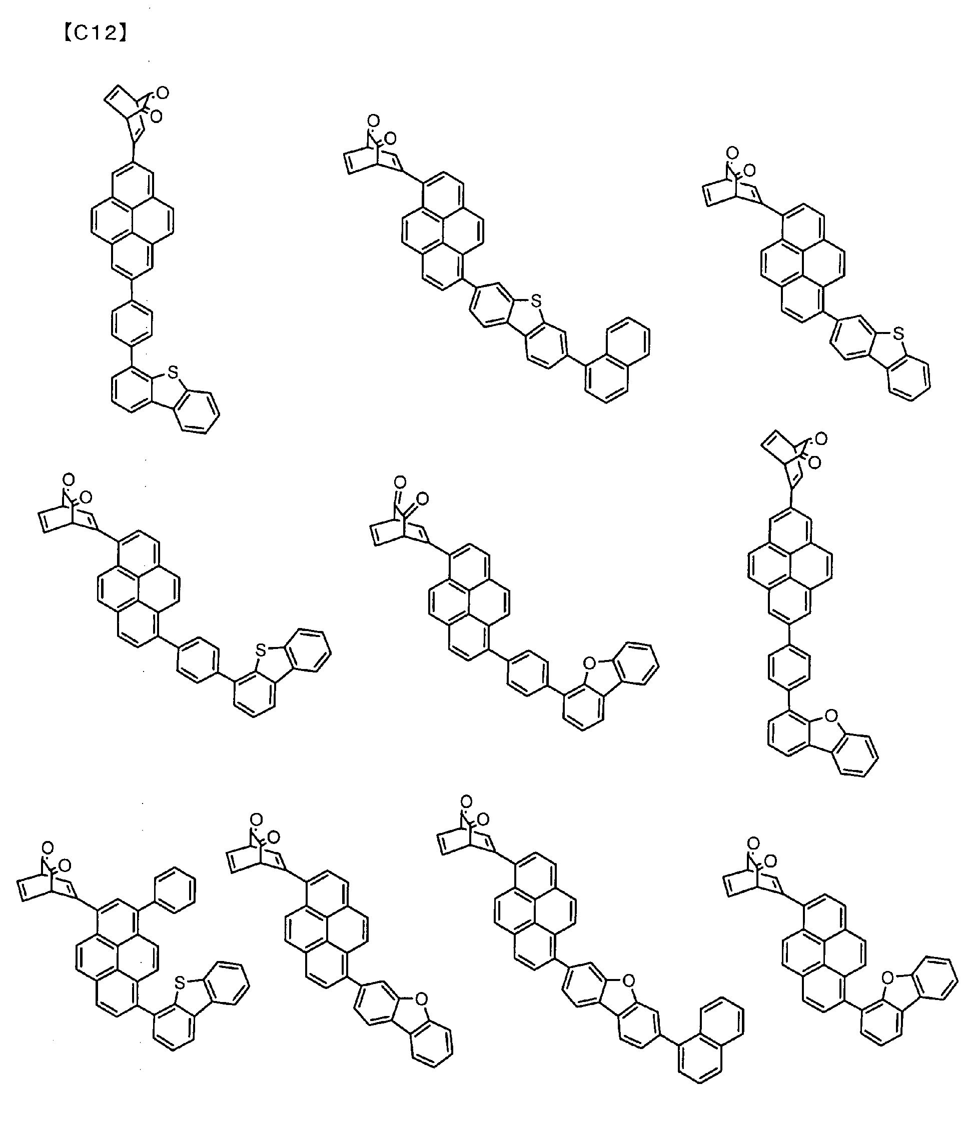

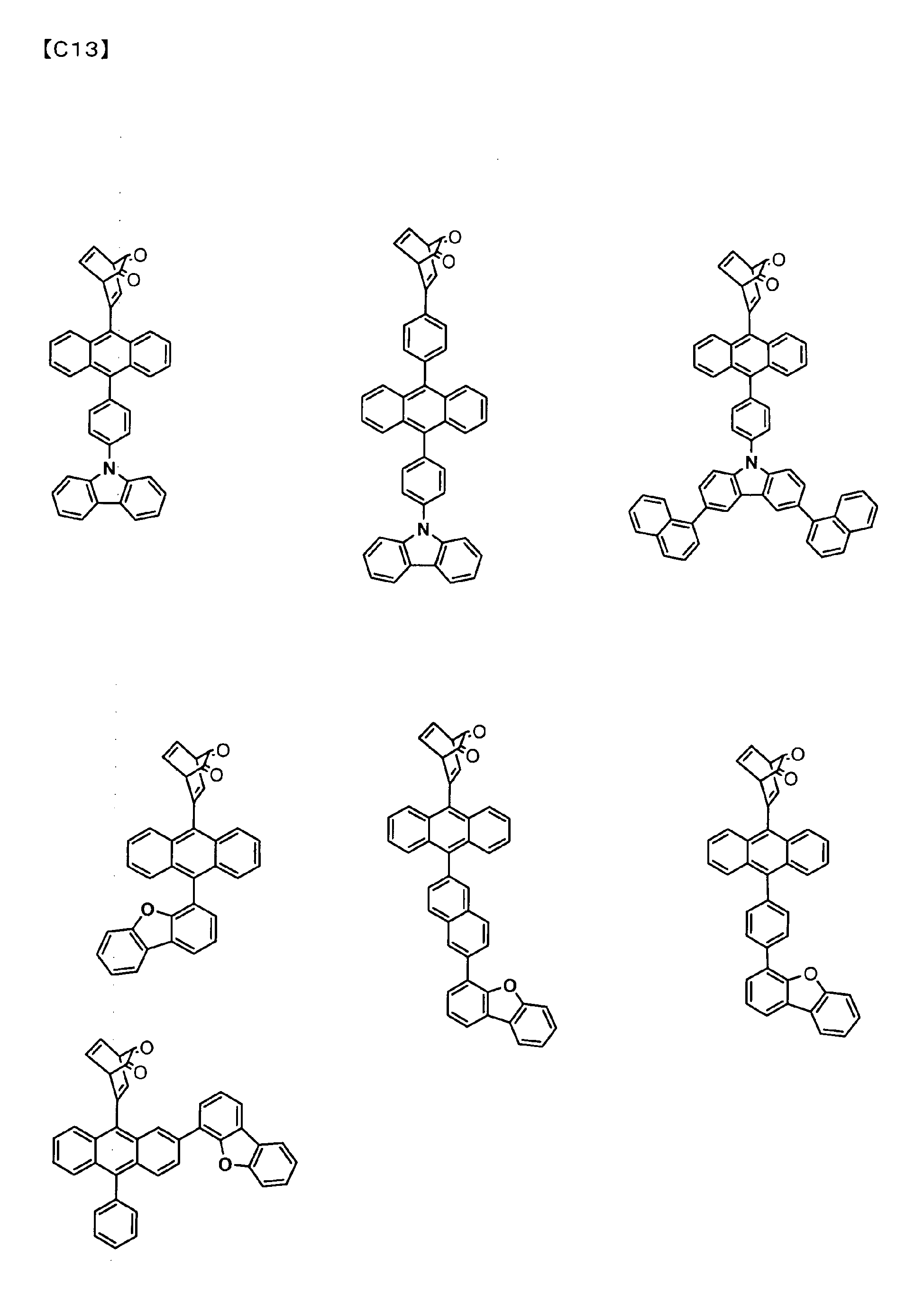

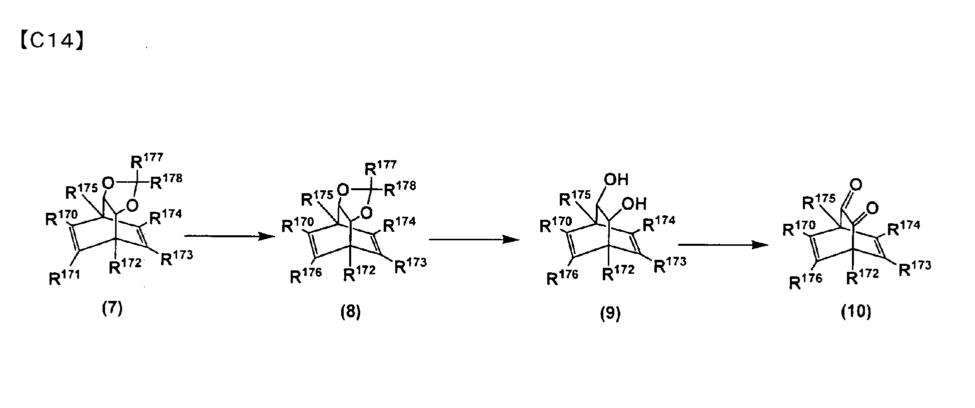

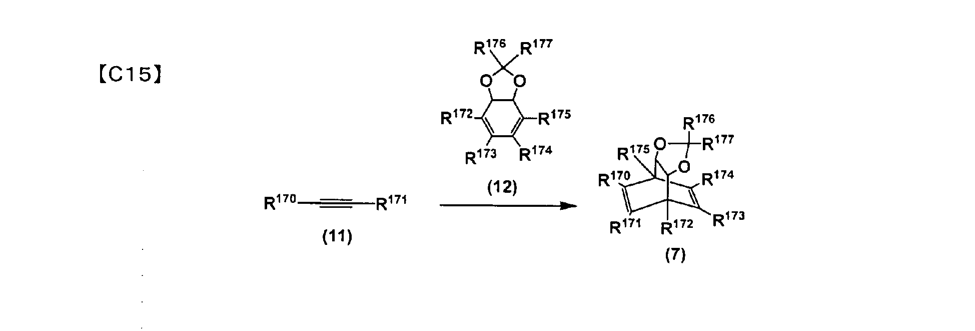

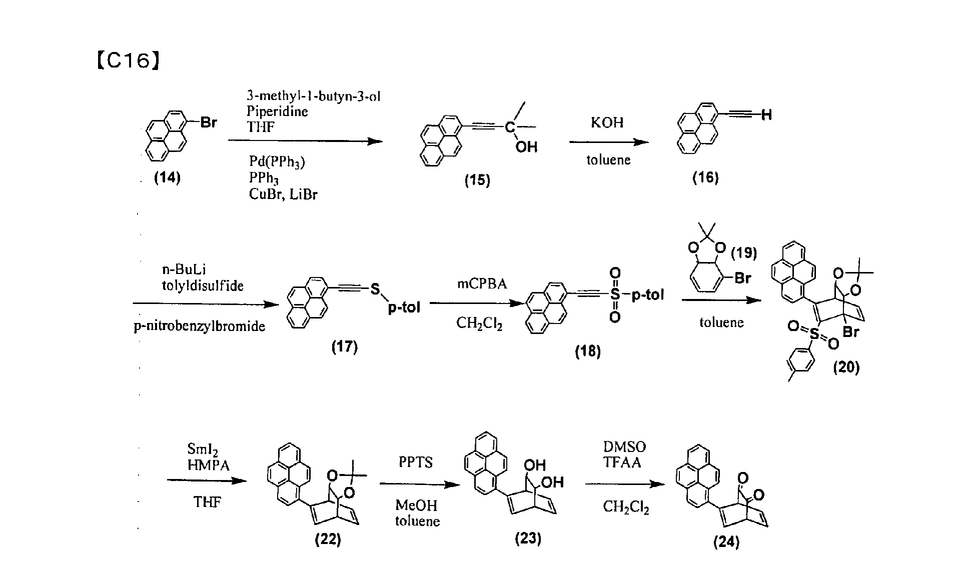

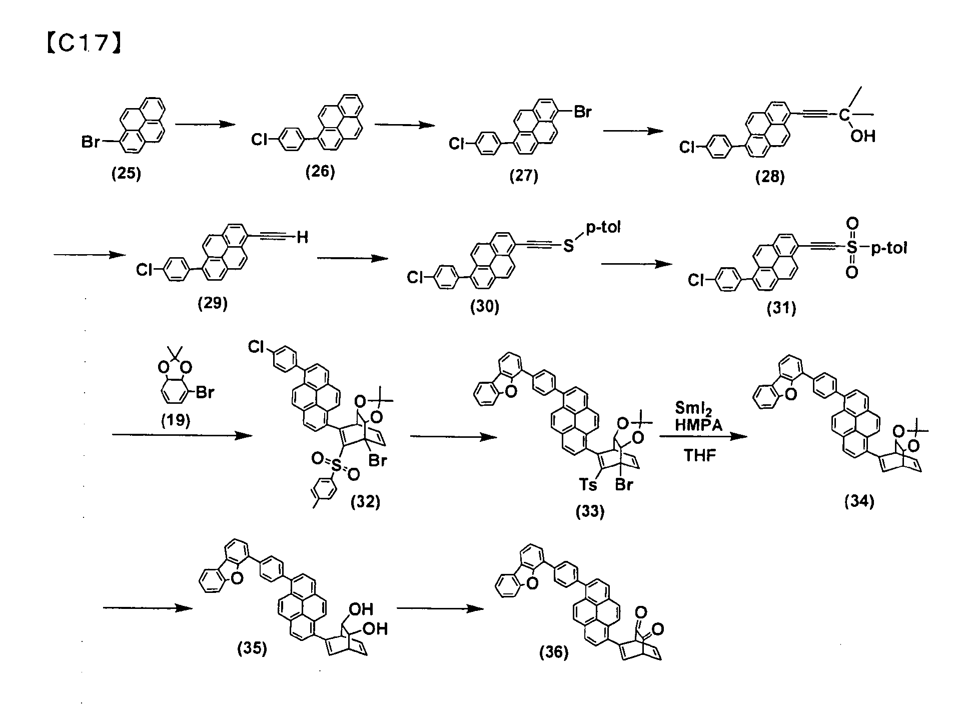

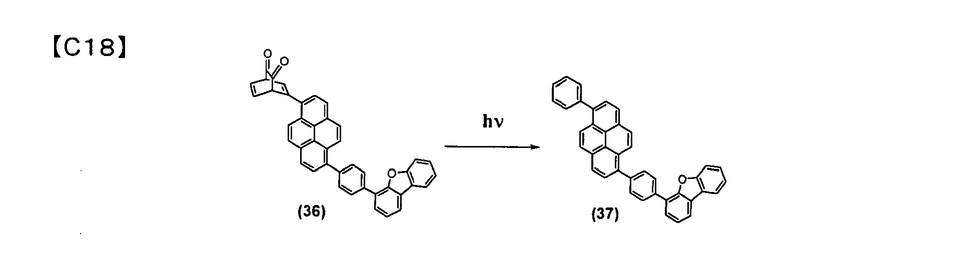

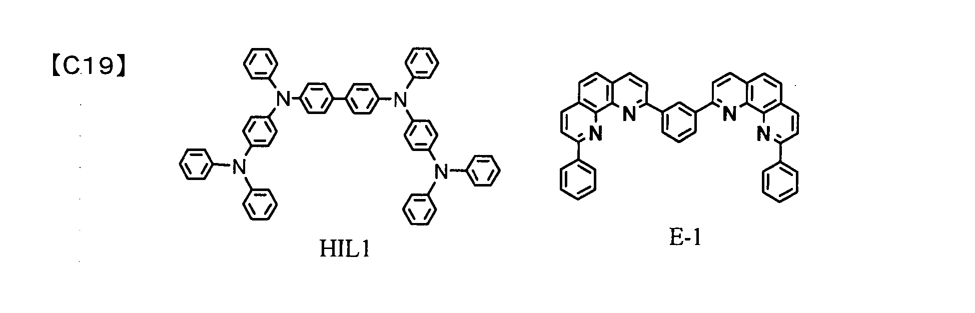

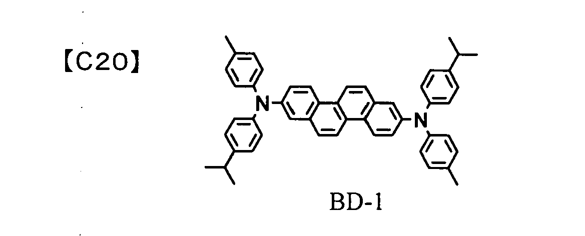

## Claims

1. A light emitting device material precursor represented by the Formula (1): 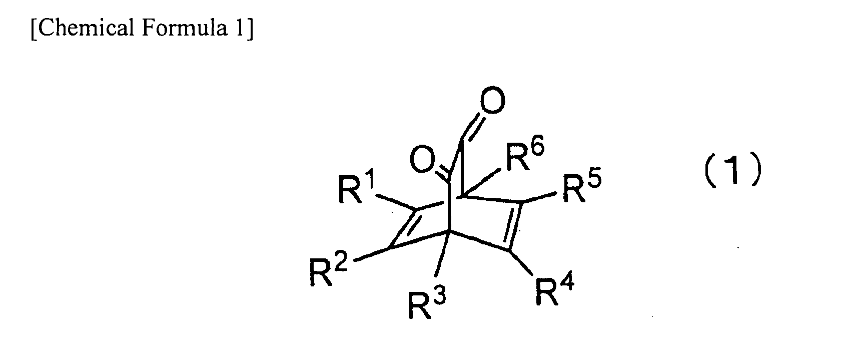 (wherein R¹ to R⁶ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups and heteroaryl groups, with the proviso that at least one of R¹ to R⁶ has a fused aromatic hydrocarbon having two or more rings).

2. A light emitting device material precursor represented by the Formula (1'): 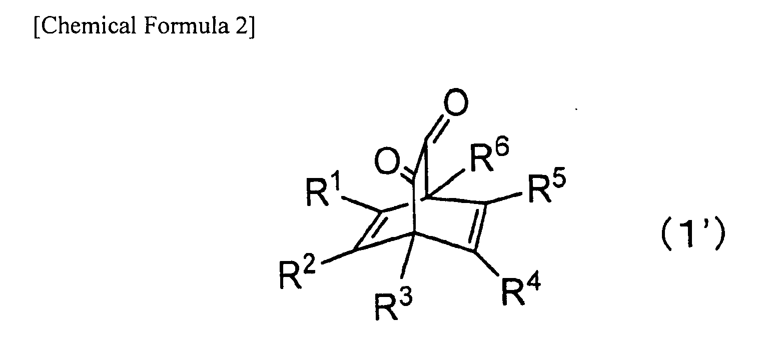 (wherein R¹ to R⁶ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups and heteroaryl groups, with the proviso that at least one of R¹ to R⁶ has a fused aromatic hydrocarbon having three or more rings).

3. The light emitting device material precursor according to claim 1, wherein at least one of R¹ to R⁶ contains a skeleton represented by the Formulae (2-1) to (2-7): 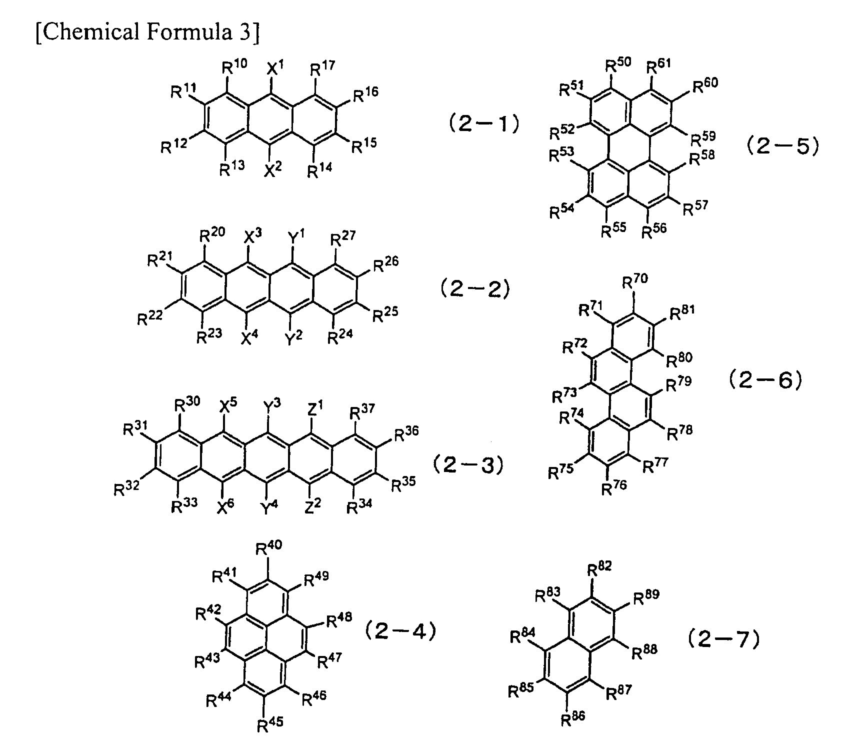 (wherein in Formulae (2-1) to (2-7), R¹⁰ to R¹⁷ R²⁰ to R²⁷, R³⁰ to R³⁷, R⁴⁰ to R⁴⁹, R⁵⁰ to R⁶¹, R⁷⁰ to R⁸¹ and R⁸² to R⁸⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused rings formed between adjacent substituents; and
X¹ to X⁶, Y¹ to Y⁴ and Z¹ to Z² each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused rings formed between adjacent substituents;
with the proviso that, in Formula (2-1), the cases where X¹ and X² are hydrogen are excluded; that, in Formula (2-2), the cases where X³ and X⁴ are hydrogen, and the cases where Y¹ and Y² are hydrogen are excluded; and that, in Formula (2-3), the cases where X⁵ and X⁶ are hydrogen, the cases where Y³ and Y⁴ are hydrogen, and the cases where Z¹ and Z² are hydrogen are excluded; and
with the proviso that at least one of R¹⁰ to R¹⁷ and X¹ to X² in Formula (2-1), at least one of R²⁰ to R²⁷, X³ to X⁴ and Y¹ to Y² in Formula (2-2), at least one of R³⁰ to R³⁷, X⁵ to X⁶, Y³ to Y⁴ and Z¹ to Z² in Formula (2-3), at least one of R⁴⁰ to R⁴⁹ in Formula (2-4), at least one of R⁵⁰ to R⁶¹ in Formula (2-5), at least one of R⁷⁰ to R⁸¹ in Formula (2-6), and at least one of R⁸² to R⁸⁹ in Formula (2-5), respectively, is used to bind to [2,2,2]-bicyclooctadiene-2,3-dione moiety in Formula (1) through direct bond or through a linking group).

4. A light emitting device material precursor represented by the Formula (3) or (4): 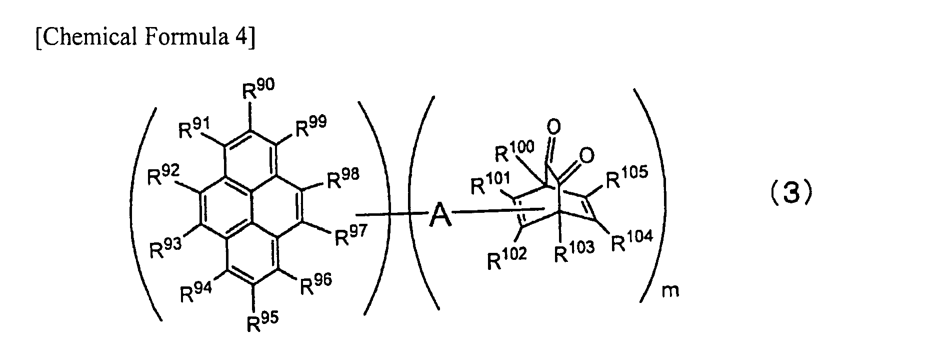 (wherein R⁹⁰ to R⁹⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents; R¹⁰⁰ to R¹⁰⁵ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens; A is selected from single bond, arylene groups and heteroarylene groups; m is an integer of 1 to 3; some of R⁹⁰ to R⁹⁹ in the number of m is used to bind to A; and at least one of R¹⁰⁰ to R¹⁰⁵ is used to bind to A). 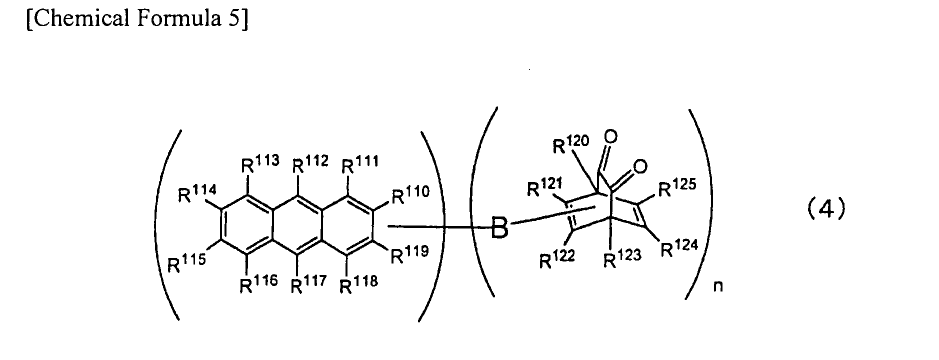 (wherein R¹¹⁰ to R¹¹⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents; R¹²⁰ to R¹²⁵ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens; B is selected from single bond, arylene groups and heteroarylene groups; n is an integer of 1 to 2; some of R¹¹⁰ to R¹¹⁹ in the number of n and one of R¹²⁰ to R¹²⁵ are used to bind to B).

5. A light emitting device material precursor represented by the Formula (5) or (6): 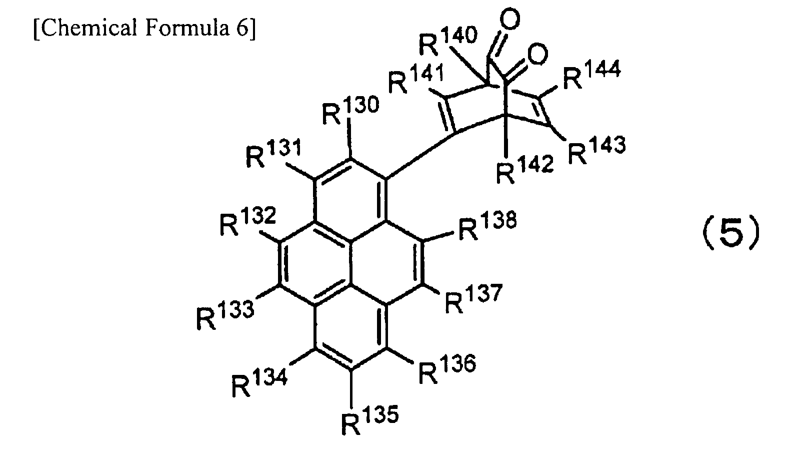 (wherein R¹³⁰ to R¹³⁸ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents; R¹⁴⁰ to R¹⁴⁴ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens.) 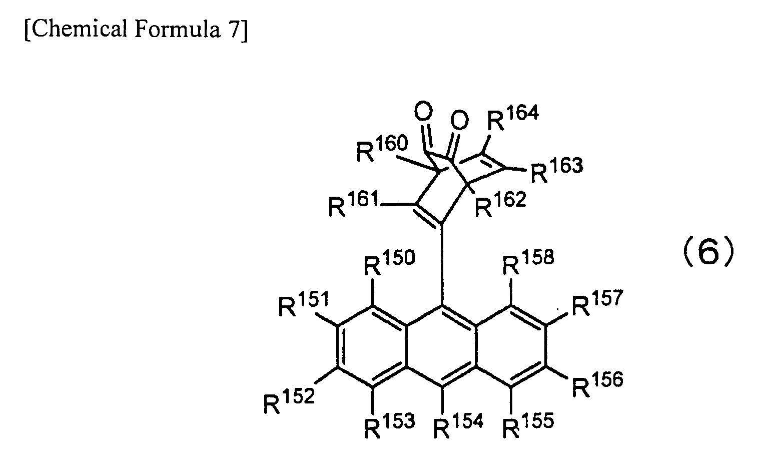 (wherein R¹⁵⁰ to R¹⁵⁸ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl groups, carboxyl group, oxycarbonyl group, carbamoyl group, amino group, silyl group, phosphineoxide group and the fused ring formed between adjacent substituents; R¹⁶⁰ to R¹⁶⁴ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens).

6. A light emitting device material precursor represented by the Formula (5') or Formula (6'): 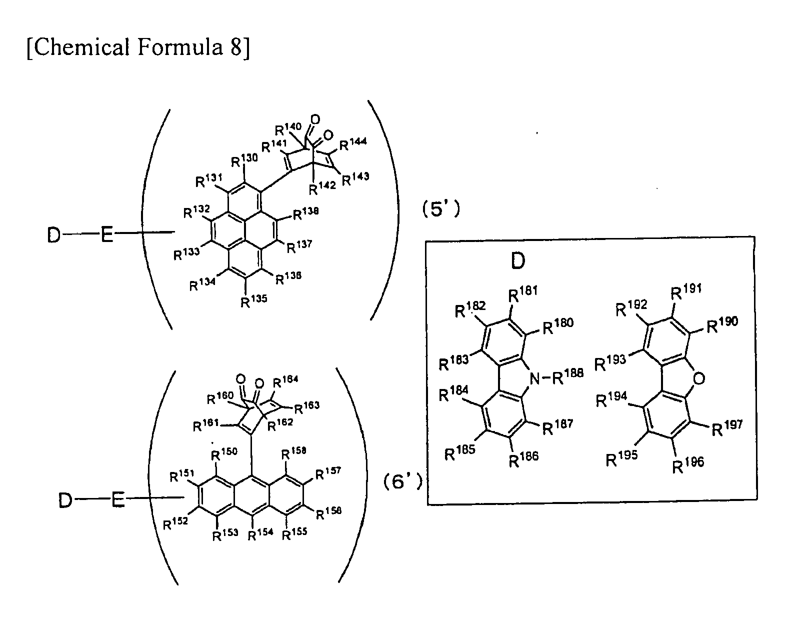 (wherein E is selected from single bond, arylene groups and heteroarylene groups; R¹⁴⁰ to R¹⁴⁴ and R¹⁶⁰ to R¹⁶⁴ each may be the same or different and is selected from hydrogen, alkyl groups, cycloalkyl groups, alkenyl groups, cycloalkenyl groups, alkoxy groups, alkylthio groups, arylether groups, aryl thioether groups, aryl groups and heteroaryl groups, with the proviso that at least one of these functional groups is used to bind to E; and
R¹⁸⁰ to R¹⁹⁷ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused ring formed between adjacent substituents, and one of these is used to bind to E).

7. A solution comprising said light emitting device material precursor according to any one of claims 1 to 6.

8. A process of producing a light emitting device, comprising the steps of coating a donor substrate with said solution containing the light emitting device material precursor according to claim 7; thereafter, converting said light emitting device material precursor to a light emitting device material; and transferring said light emitting device material on said donor substrate to a device substrate.

9. The process of producing a light emitting device, according to claim 7, wherein means for conversion to the light emitting device material is light irradiation.

10. A light emitting device comprising a lower electrode, a light emitting device material layer having at least one layer composed of a light emitting device material, and an upper electrode, said layer composed of a light emitting device material containing said light emitting device material precursor according to any one of claims 1 to 6 at a content of less than 5%.

11. A process of producing a bicyclo-[2,2,2]-cyclooctadiene-2,3-dione derivative, comprising the steps of obtaining a compound represented by the Formula (8) from a compound represented by the Formula (7) below by a reaction replacing R¹⁷¹; eliminating R¹⁷⁷ to R¹⁷⁸ which are protective groups in the obtained compound represented by the Formula (8) to obtain a compound represented by the Formula (9); and converting the compound represented by the Formula (9) to a compound represented by the Formula (10) by an oxidation reaction of the compound represented by the Formula (9);
(wherein R¹⁷⁰ is an aryl group or heteroaryl group; R¹⁷¹ is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and substituted sulfonyl groups; R¹⁷² to R¹⁷⁶ are selected from hydrogen, alkyl groups, cycloalkyl groups, alkenyl groups, cycloalkenyl groups, alkoxy groups, alkylthio groups, arylether groups, aryl thioether groups, aryl groups, heteroaryl groups and halogens; R¹⁷⁷ to R¹⁷⁸ is selected from hydrogen, alkyl groups, alkoxy groups, aryl groups and arylether groups).

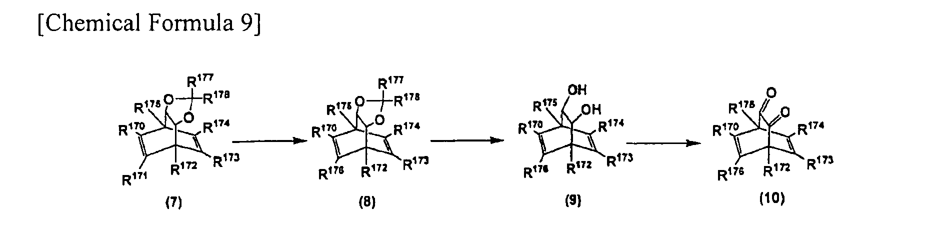

12. The process of producing a bicyclo-[2,2,2]-cyclooctadiene-2,3-dione derivative, according to claim 10, wherein R¹⁷¹ is an electron-withdrawing group.

13. The process of producing a bicyclo-[2,2,2]-cyclooctadiene-2,3-dione derivative, according to claim 11 or 12, wherein R¹⁷⁰ is a functional group containing any one of the skeleton represented by the Formulae (2-1) to (2-7). 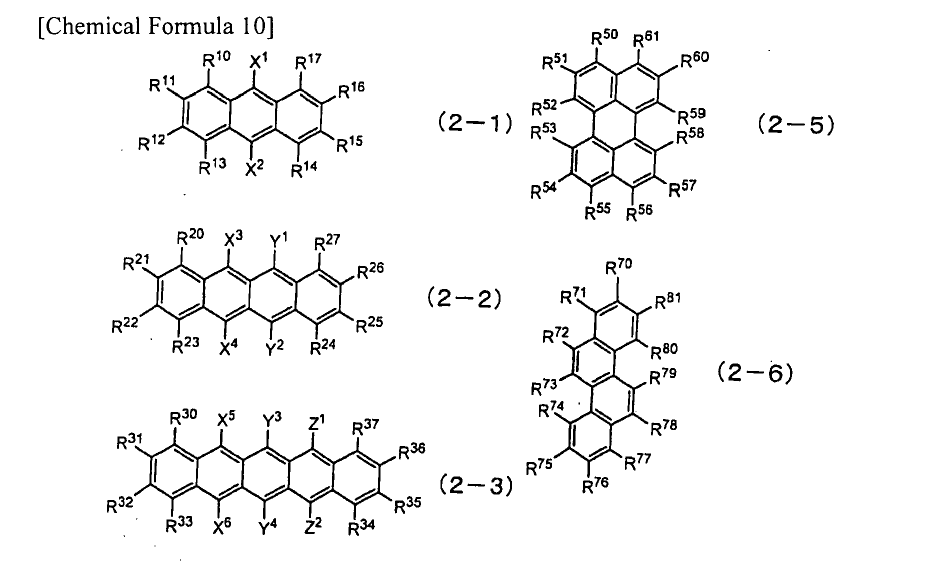 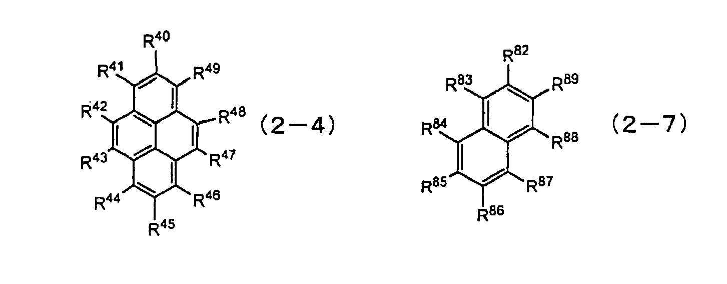 (wherein in Formulae (2-1) to (2-7), R'° to R¹⁷, R²⁰ to R²⁷, R³⁰ to R³⁷, R⁴⁰ to R⁴⁹, R⁵⁰ to R⁶¹, R⁷⁰ to R⁸¹ and R⁸² to R⁸⁹ each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused rings formed between adjacent substituents; and
X¹ to X⁶, Y¹ to Y⁴ and Z¹ to Z² each may be the same or different and is selected from hydrogen, alkyl groups, cycloalkyl groups, heterocyclic groups, alkenyl groups, cycloalkenyl groups, alkynyl groups, alkoxy groups, alkylthio groups, arylether groups, aryl thioether groups, aryl groups, heteroaryl groups, halogens, cyano group, carbonyl group, carboxyl group, oxycarbonyl group, carbamoyl group, amino group, silyl group, phosphineoxide group and fused rings formed between adjacent substituents;
with the proviso that, in Formula (2-1), the cases where X¹ and X² are hydrogen are excluded; that, in Formula (2-2), the cases where X³ and X⁴ are hydrogen, and the cases where Y¹ and Y² are hydrogen are excluded; and that, in Formula (2-3), the cases where X⁵ and X⁶ are hydrogen, the cases where Y³ and Y⁴ are hydrogen, and the cases where Z¹ and Z² are hydrogen are excluded.
